(19) Europäisches Patentamt
European Patent Office
Office européen des brevets

(11) EP 2 138 584 B1

(12) # EUROPEAN PATENT SPECIFICATION

(45) Date of publication and mention of the grant of the patent:
**14.08.2013 Bulletin 2013/33**

(51) Int Cl.:
***C12N 15/867*** (2006.01) ***A61K 48/00*** (2006.01)

(21) Application number: **08011698.1**

(22) Date of filing: **27.06.2008**

(54) **Foamy viral envelope genes**

Foamyvirale Hüllgene

Gènes à enveloppe virale mousseuse

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**30.12.2009 Bulletin 2009/53**

(73) Proprietor: **Vectoria Forschungsförderungs Verein e.V.**
**97078 Würzburg (DE)**

(72) Inventors:
- **Lindemann, Dirk**
  **01239 Dresden (DE)**
- **Stirnnagel, Kristin**
  **01099 Dresden (DE)**
- **Lüftenegger, Daniel**
  **91052 Erlangen (DE)**

(74) Representative: **Elbel, Michaela**
**Pateris**
**Patentanwälte, Partnerschaft**
**Postfach 33 07 11**
**80067 München (DE)**

(56) References cited:
**EP-A- 0 864 652 US-B1- 6 277 601**

- **STANKE NICOLE ET AL: "Ubiquitination of the prototype foamy virus envelope glycoprotein leader peptide regulates subviral particle release" JOURNAL OF VIROLOGY, vol. 79, no. 24, December 2005 (2005-12), pages 15074-15083, XP002509102 ISSN: 0022-538X**
- **DUDA ANJA ET AL: "Characterization of the prototype foamy virus envelope glycoprotein receptor-binding domain" JOURNAL OF VIROLOGY, vol. 80, no. 16, August 2006 (2006-08), pages 8158-8167, XP002509103 ISSN: 0022-538X**

Remarks:
The file contains technical information submitted after the application was filed and not included in this specification

EP 2 138 584 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the invention

The present invention concerns the technical field of nucleic acids and expression-optimized nucleic acids. The present invention especially concerns nucleic acids comprising a mutated foamy viral envelope gene encoding a foamy viral envelope polypeptide, which comprises a leader peptide (LP), a surface unit (SU) and a transmembrane domain (TM). The present invention also relates to modified polypeptides encoded by these nucleic acids. Furthermore, the present invention regards a method for preparing pseudotyped vector particles as well as a method for treating a genetic disorder comprising administering a nucleic acid or a polypeptide encoded by that nucleic acid.

### Background of the invention

Gene therapy is used to insert genes into a patient's cells or tissues to treat hereditary diseases, whereby a defective mutant allele can be replaced by a functional one. Though the technology is still in its beginning and has been used with little success, it is promising for the future. However, numerous problems exist that impede gene therapy using viral vectors, such as undesired side effects. For example, it has to be ensured that the virus will infect the correct cellular target and that the inserted gene does not disrupt any vital genes in the human genome. If the transduced gene is inserted into genes regulating cell division uncontrolled cell growth, i.e. cancer can occur by activation of oncogenes (Li et al., 2002; Check, 2005). Gene therapy trials to treat SCID were halted or restricted in the USA, when leukemia was reported in three of eleven patients treated in the French Therapy X-linked SCID gene therapy trial.

The foamy virus subgroup of retroviruses has attracted scientific interest, because of their unique replication strategy and their potential use as gene transfer vectors (Weiss, 1996). It has been proposed that foamy viruses may be ideal tools for the development of a gene delivery system, due to specific properties of this virus group, such as the benign course of natural foamy viral infections, their very broad host cell range, and an extended packaging limit, due to the size of the foamy virus genome (Russel and Miller, 1996; Schmidt and Rethwilm, 1995; US 5,929,222; US 6,111,087). However, limited availability of improved foamy virus envelope genes has so far not allowed developing methods for preparing pseudotyped viral vectors that efficiently transfer genes into a wide variety of cell types. Therefore, there is a demand for new nucleic acids, polypeptides and methods that improve efficiency of preparing pseudotyped vector particles and improve efficiency of transduction.

### Summary of the invention

The present invention concerns an nucleic acid comprising a foamy viral envelope gene encoding a foamy viral envelope polypeptide, the nucleic acid is expression-optimized the foamy viral envelope gene comprises at least one mutation, which leads on expression of the mutated foamy viral envelope gene to a modified envelope polypeptide, which comprises at least one inactivated ubiquitination site in the leader peptide, the modified envelope polypeptide pseudotypes a viral vector for infecting at least one host cell, wherein the foamy viral envelope gene is selected from the group consisting of SEQ ID NO: 7 (PE01), SEQ ID NO: 8 (PE02), SEQ ID NO: 9 (PE03), SEQ ID NO: 10 (SE01), SEQ ID NO: 11 (SE02) and SEQ ID NO: 12 (SE03). The infectivity of the pseudotyped viral vector is increased by expression-optimization up to about 50-fold compared to a non-optimized nucleic acid encoding the same modified envelope polypeptide.

In addition, the present invention is directed to a method for preparing at least one pseudotyped vector particle comprising the steps of:

a) providing at least one cell;

b) adding to the cell a non-foamy viral vector and a nucleic acid, which comprises a foamy viral envelope gene encoding a foamy viral envelope polypeptide, the foamy viral envelope gene comprises at least one mutation, which leads on expression of the mutated foamy viral envelope gene to a modified envelope polypeptide, which comprises at least one inactivated ubiquitination site; and

c) harvesting at least one pseudotyped vector particle produced by the cell.

Further, the invention relates to a method for preparing at least one pseudotyped vector particle comprising the steps of:

a) providing at least one cell;

b) adding to the cell at least one viral vector and an expression-optimized nucleic acid comprising a foamy viral envelope gene encoding a foamy viral envelope polypeptide, the foamy viral envelope gene comprises at least one mutation, which leads on expression of the mutated foamy viral envelope gene to a modified envelope polypeptide, which comprises at least one inactivated ubiquitination site, the modified envelope polypeptide pseudotypes the viral vector to prepare the pseudotyped vector particle for infecting the host cell; the infectivity of the pseudotyped viral vector particle is increased by expression-optimization up to about 50-fold compared to a non-optimized nucleic acid encoding the same modified envelope polypeptide; and

c) harvesting at least one pseudotyped vector particle produced by the cell.

The present invention also concerns a method for treating a genetic disorder comprising administering to a subject a nucleic acid comprising a foamy viral envelope gene comprising at least one mutation, wherein the mutation leads on expression of the mutated foamy viral envelope gene to a modified envelope polypeptide, which comprises at least one inactivated ubiquitination site.

Furthermore, the invention relates to a method for treating a genetic disorder comprising administering to a subject a polypeptide encoded by a foamy viral envelope gene comprising at least one mutation, wherein the mutation leads on expression of the mutated foamy viral envelope gene to a modified envelope polypeptide, which comprises at least one inactivated ubiquitination site.

In addition, the invention concerns the use of an nucleic acid for the manufacture of a drug for the treatment and/or prevention of a genetic disorder, virally induced disease or cancer, the nucleic acid is expression-optimized and comprises a foamy viral envelope gene, which comprises at least one mutation, which leads on expression of the mutated foamy viral envelope gene to a modified envelope polypeptide, which comprises at least one inactivated ubiquitination site in the leader peptide, the modified envelope polypeptide pseudotypes a viral vector for infecting at least one host cell, wherein the foamy viral envelope gene is selected from the group consisting of SEQ ID NO: 7 (PE01), SEQ ID NO: 8 (PE02), SEQ ID NO: 9 (PE03), SEQ ID NO: 10 (SE01), SEQ ID NO: 11 (SE02) and SEQ ID NO: 12 (SE03). The infectivity is increased by expression-optimization up to about 50-fold compared to a non-optimized nucleic acid encoding the same modified envelope polypeptide.

**Brief description of the figures**

Fig. 1 A shows the putative membrane topology of a precursor and a mature foamy viral envelope polypeptide (env) inserted into the cell membrane. Fig. 1 B shows the domain organization of a foamy viral env including LP (glycoprotein (gp) 18), SU (gp80) and TM (gp48) according to the prior art.

Fig. 2 displays a pcz human foamy viral vector (HFV) with a coding domain of env EM167.

Fig. 3 displays a pci simian foamy viral (SFV) 1 vector with a coding domain of env SM05.

Fig. 4 shows wild type (wt) env and a selection of its different modifications. The encoding env genes were cloned in pczHFV or pciSFV-1 vectors as indicated. Amino acids resulting from a mutation are indicated as R instead of K. The open rectangle is a replacement character for sequences that are not displayed. Y indicates glycosylation.

In Figs. 5 and 6 absolute and relative infectivities of vectors including wt env and modified env compared to vectors including vesicular stomatitis virus G protein (VSV-G) are shown. Env was used to pseudotype prototype foamy virus (PFV), murine leukemia virus (MLV) and human deficiency virus 1 (HIV-1). Relative infectivities are normalized to VSV-G for MLV and HIV-1 and to PVF env wt for PFV vectors, and absolute infectivities are indicated as focus-forming units per ml (ffu/ml) by means of enhanced green fluorescent protein (EGFP).

In Figs. 7 and 8 absolute and relative infectivities of vectors including wt env, modified env and expression-optimized env compared to vectors including VSV-G are shown.

Fig. 9 shows a wt env gene and a selection of its different modified env comprising deletions.

In Figs. 10 and 11 absolute and relative infectivities of vectors including wt env, modified env comprising deletions compared to vectors including VSV-G are shown.

Fig. 12 shows a wt env gene and a selection of its different modified env comprising glycine-serine linked fluorescent proteins.

In Figs. 13 and 14 absolute and relative infectivities of vectors including wt env, modified env and modified env comprising fluorescent proteins compared to vectors including VSV-G are shown.

Fig. 15 exemplifies a selection of vectors used among others in the invention for preparing pseudotyped vector particles.

Fig. 16 exemplifies a selection of expression constructs used in the invention for preparing pseudotyped vector particles.

Fig. 17 compares titers of vector particles as transfection results with and without serum application.

Fig. 18 displays titers of enveloped vector particles carrying a therapeutically useful gene, MGMT, before and after concentrating by centrifugation using foamy viral env EM140 and SM04.

Fig. 19 displays titers of vector particles enveloped and pseudotyped by env EM140 compared to VSV-G before and after concentrating by centrifugation.

Figs. 20 and 21 show rates of gene transfer in human CD34+ cells after transduction with viral vectors comprising env EM140 or VSV-G. In Fig. 20 the gene transfer rate is indicated by GFP positive cells measured by fluorescent activated cell sorting (FACS), and in Fig. 21 the gene transfer rate is indicated by GFP positive colonies measured by a progenitor assay.

Fig. 22 depicts the efficiency of transduction in marmoset CD34+ cells with VSV-G and env EM140 5 and 9 days after transduction measured by FACS.

Fig. 23 displays the transduction efficiency of various foamy viral vectors enveloped by env EM140 depending on the type of target cell line used.

Fig. 24 demonstrates the effect of the transferred MGMTp140k gene in K562 cells after treatment with 1,3-bis-(2-chloroethyl)-1-nitrosourea (BCNU).

Fig. 25 gives an overview of the domain organization of foamy viral env and an expanded view of different N terminal TM sequences of env derived from foamy viruses infecting different hosts, like cats, cattle, horses etc.. Homologue lysine residues of env are indicated by K.

**Detailed description of the invention**

A nucleic acid is described, comprising a foamy viral envelope gene encoding a foamy viral envelope polypeptide, which comprises leader peptide (LP), surface unit (SU) and a transmembrane domain (TM), the foamy viral envelope gene comprises at least one mutation, which leads on expression of the mutated foamy viral envelope gene to a modified envelope polypeptide, which comprises at least one inactivated ubiquitination site, wherein the at least one inactivated ubiquitination site is located at the transmembrane domain of the modified envelope polypeptide.

The term "nucleic acid" as used herein refers to a macromolecule composed of monomeric nucleotides, wherein nucleic acids include amongst others deoxynucleic acids (DNA); ribonucleic acids (RNA); artificial nucleic acids. The term "envelope gene" as used herein refers to a foamy viral sequence encoding an envelope polypeptide (env), which is a glycoprotein that forms prominent surface spikes within the viral envelope. Env is a trimeric complex with a highly unusual biosynthesis among retroviral glycoproteins. It is translated as a full length precursor protein into the rough endo-plasmatic reticulum (ER) and initially has a type II protein conformation with both its N and C termini located intracytoplasmic (Fig.1A). During its transport to the cell surface it is postranslationally processed by cellular proteases into three subunits. Env is responsible for the foamy viral host tropism and is also found on the surface of transduced cells. Binding of env to a specific receptor on the host cell's membrane leads to a conformational change of env and finally to a fusion of viral envelope and cell membrane. In contrast to other retroviral envelope proteins, foamy viral env is essential for budding, it carries an ER-retrieval signal for retention in the ER, and its LP is not processed by a signal peptide complex, but by a furin or furin like protease. A foamy viral env is an envelope polypeptide from a foamy virus or an envelope polypeptide derived from an envelope polypeptide from a foamy virus. Fig. 1A shows the putative membrane topology of the precursor and the mature env inserted into the cell membrane. Fig. 1 B shows the domain organization of PFV env with LP, SU and TM from amino acid position 1 (AS 1) to 988. The term "leader peptide" (LP) as used herein refers to a foamy viral N terminal signal sequence that comprises two polar domains and an intermediate hydrophobic domain. In contrast to other retroviral signal sequences, LP is essential for morphogenesis, infectivity and release of vector particles, wherefore coexpressed env and gag interact specifically with each other via a so-called budding region in LP. The term "surface unit" (SU) as used herein refers to a domain of env that is located at an exposed position at a cell surface. The SU comprises the major immunogenic epitope responsible for receptor specific binding of a foamy viral vector particle to a host cell. The term "transmembrane domain" (TM) as used herein refers to a domain of env that completely or partly spans a biological membrane to anchor the peptide within the membrane and to retain the peptide within the cell.

The term "mutation" as used herein refers to a change or an amendment of a particular nucleotide sequence of a nucleic acid. The mutation includes small-scale mutations, like point mutations, single nucleotide exchanges, insertions, deletions as well as large-scale mutations, like amplifications, deletions of larger regions, translocations and inversions. Included are also loss- and gain-of-function mutations, antimor-phic, lethal and conditional mutations. The term "ubiquitination" as used herein refers to a posttranslational covalent linking of a polypeptide to ubiquitin by a lysine-dependent or lysine-independent process. Ubiquitin can be linked as a monomer, as oligomeric chains or cross-linked oligomers. The process of ubiquitination can comprise an activation of ubiquitin, its transfer to the binding site, and its linking to the polypeptide. The ubiquitination site is the location of the polypeptide, where ubiquitin can be linked to, like a lysine residue. The term "ubiquitination site" also comprises locations, where ubiquitin can potentially be coupled to, but physiologically no ubiquitination occurs, e.g. the ubiquitination sites in TM env (Stanke et al., 2005). If the ubiquitination site of a polypeptide is inactivated, it is no longer possible to couple ubiquitin to this polypeptide. This can be due to a failure in the mechanism of the ubiquitination or preferably the lack of a binding site or a specific binding site. The function of ubiquitination is manifold. It can be a signal for polypeptide degradation, preservation or a label for polypeptide endocytosis. The term "polypeptide" as used herein refers to a polymer that comprises multiple amino acids linked by peptide bonds regardless of length and conformation.

In Stanke et al. (2005) the role of certain modified env in glycoprotein function and foamy viral replication is investigated. The modified env of this publication show inactivated ubiquitination sites within the LP. In contrast to Stanke et al. (2005), the modified env according to the invention shows inactivated ubiquitination sites in the TM of the polypeptides. Stanke et al. (2005) describe that no ubiquitination of other PFV env domains than LP is observed. In particular, for TM containing at least four additional lysine residues, i.e. potential ubiquitination sites in its C terminal cytoplasmic tail no ubiquitination can be shown. LP and TM are different domains of env that are located at antipodal ends of the precursor polypeptide. The LP has a type II conformation, whereas the TM subunit has a type I conformation and associates with the SU on the luminal side. For a foamy viral budding process the contact of LP containing an essential, conserved WXXW sequence motif with the N terminus of the foamy viral gag protein is an essential interaction (Wilk et al., 2001; Lindemann at al., 2001). Most probably due to the crucial interaction between capsid and foamy viral env LP, this cleavage product is particle associated. In contrast to LP, TM is responsible for fusion with the host’s membrane, anchoring within the viral membrane as well as retrieval and retention in the ER. Therefore, these findings demonstrate the functional uniqueness of LP. In spite of Stanke et al. (2005) describe no ubiquitination of other PFV env domains than LP during budding, the inventors were surprisingly able to considerably improve infectivity of viral vectors enveloped or pseudotyped by foamy viral env according to the invention, wherein at least one ubiquitination site of env is inactivated at TM. The modified env according to the invention, which comprises at least one inactivated ubiquitination site located at the TM of the modified envelope polypeptide were used to pseudotype different types of vectors that were applied to eukaryotic cells. The measured infectivities of these vectors pseudotyped by modified env were increased due to the inactivated ubiquitination site located at the TM of the modified env (Figs. 5 and 6).

In a preferred embodiment of the invention the mutation of the foamy viral envelope gene, which leads on expression of the mutated foamy viral envelope gene to a modified envelope polypeptide, which comprises at least one inactivated ubiquitination site located at the transmembrane domain of the modified envelope polypeptide, leads on expression of the foamy viral envelope gene to a lysine exchange in the envelope polypeptide. In a further preferred embodiment of the invention lysine is exchanged against an amino acid selected from the group consisting of alanine, histidine, glycine and arginine. Arginine and histidine are positively charged amino acids like lysine. Glycine and alanine posses uncharged side chains. The exchange of the amino acid lysine against another amino acid removes the lysine binding site of ubiquitin, whereby the ubiquitination site is inactivated, i.e. the env cannot be ubiquitinated at these exchanged amino acid sites. On the level of the env gene all possible base triplets encoding lysine can be exchanged with all possible base triplets encoding arginine, glycine, histidine and alanine.

The counting of the amino acids begins with amino acid position 1 at the N terminus of the polypeptide and ends with the highest amino acid position, e.g. in Fig. 1 B with position 988, at the C terminus of the polypeptide. In the most preferred embodiment of the invention the mutation leads on expression of the foamy viral envelope gene to a modification of the envelope polypeptide at an amino acid position including 951, 952, 955, 959, 960, 961, 962, 968, 969, 972, 976 to 982 and 984 to 988. In another preferred embodiment of the invention the mutated foamy viral envelope gene, which leads on expression to a modified envelope polypeptide, which comprises at least one inactivated ubiquitination site located at TM of the modified envelope polypeptide, comprises at least one second mutation, which is located at the LP of the modified envelope polypeptide.

In a further preferred embodiment the second mutation leads on expression of the foamy viral envelope gene to a modification of the envelope polypeptide at an amino acid position selected from the amino acid positions 14 to 55, and amino acid position 1 is the first amino acid of the N terminus of the envelope polypeptide. In the most preferred embodiment of the invention the second mutation leads on expression of the foamy viral envelope gene to a modification of the envelope polypeptide at an amino acid position selected from the group consisting of amino acid positions 14, 15, 18, 34, 53 and 55.

In another preferred embodiment of the invention the mutated foamy viral envelope gene, which leads on expression to a modified envelope polypeptide, which comprises at least one inactivated ubiquitination site located at TM of the modified envelope polypeptide, comprises a sequence selected from the group consisting of SEQ ID NO: 8 (PE02), SEQ ID NO: 9 (PE03), SEQ ID NO: 11 (SE02) and SEQ ID NO: 12 (SE03). The mutated foamy viral envelope genes, which comprise a sequence selected from the group consisting of SEQ ID NO: 8 (PE02), SEQ ID NO: 9 (PE03), SEQ ID NO: 11 (SE02) and SEQ ID NO: 12 (SE03), encode the corresponding modified polypeptide selected from the group consisting of env PE02, env PE03, env SE02 and env SE03. The modified env of this preferred embodiment as well as the corresponding wt env are shown in Fig. 2 encoded by mutated HFV env genes and SFV-1 env genes. In env PE02 and env PE03 the mutations cause modifications in TM of the expressed env at amino acid positions 984 to 986, also called ER modifications, and/or at amino acid positions 959 and 976, also called ER+ modifications. In env SE02, env SE03, the mutations cause modifications in TM of the expressed env at amino acid positions 985, 987 and 988, also called ER modifications, and/or at amino acid positions 960 and 977, also called ER+ modifications. HFV env PE02 and env PE03 possess additional modifications in LP at amino acid positions 14, 15, 18, 34 and 53 similar as in env EM140, and SFV- 1, env SE02 and env SE03 possess additional modifications in LP at amino acid positions 14, 15, 34, 53 and 55 similar as in env SM04. Modifications in LP are also called Ubi modifications. Env EM002 is the human wt polypeptide, and env wt is the simian wt polypeptide.

A polypeptide is described, encoded by the nucleic acid comprising a foamy viral envelope gene encoding a foamy viral envelope polypeptide, which comprises a leader peptide, a surface unit and a transmembrane domain, the foamy viral envelope gene comprises at least one mutation, which leads on expression of the mutated foamy viral envelope gene to a modified envelope polypeptide, which comprises at least one inactivated ubiquitination site, wherein the at least one inactivated ubiquitination site is located at the transmembrane domain of the modified envelope polypeptide. A nucleic acid is described, comprising a foamy viral envelope gene encoding a foamy viral envelope polypeptide, which comprises a leader peptide, a surface unit and a transmembrane domain, the foamy viral envelope gene comprises at least one mutation, which leads on expression of the mutated foamy viral envelope gene to a modified envelope polypeptide, which comprises at least one inactivated ubiquitination site at the leader peptide, wherein the modified envelope polypeptide further comprises at least one deletion at the N terminus. The term "deletion" as used herein refers to a loss of a part of a polypeptide. The modified env according to the invention, which comprises the deletion were used to pseudotype different types of vectors that were applied to eukaryotic cells. The measured infectivities of these vectors pseudotyped by env comprising the deletion are increased significantly due to the deletion (Figs. 10 and 11). A polypeptide is described, encoded by the nucleic acid comprising a foamy viral envelope gene encoding a foamy viral envelope polypeptide, which comprises a leader peptide, a surface unit and a transmembrane domain, the foamy viral envelope gene comprises at least one mutation, which leads on expression of the mutated foamy viral envelope gene to a modified envelope polypeptide, which comprises at least one inactivated ubiquitination site at the leader peptide, wherein the modified envelope polypeptide further comprises at least one deletion at the N terminus. The modified env comprising a deletion can be expressed in an expression system or can be synthesized artificially.

The present invention is directed to an nucleic acid comprising a foamy viral envelope gene encoding a foamy viral envelope polypeptide, the nucleic acid is expression-optimized, the foamy viral envelope gene comprises at least one mutation, which leads on expression of the mutated foamy viral envelope gene to a modified envelope polypeptide, which comprises at least one inactivated ubiquitination site in the leader peptide, the modified envelope polypeptide pseudotypes a viral vector for infecting at least one host cell, wherein the foamy viral envelope gene is selected from the group consisting of SEQ ID NO: 7 (PE01), SEQ ID NO: 8 (PE02), SEQ ID NO: 9 (PE03), SEQ ID NO: 10 (SE01), SEQ ID NO: 11 (SE02) and SEQ ID NO: 12 (SE03). The infectivity of the pseudotyped viral vector is increased by expression-optimization up to about 50-fold compared to a non-optimized nucleic acid encoding the same modified envelope polypeptide. The term "expression-optimized" as used herein refers to an alteration of genetic code, codons in the gene or coding regions of nucleic acids, so that the genetic code and the codons are more suitable for the expression system of the host organism, e.g. primates, without altering the amino acids encoded by the nucleic acid. The term "expression-optimized" includes among others codon-optimization, i.e. a gene’s frequency of codon usage is designed to mimic the frequency of preferred codon usage of the host cell. The term also includes avoiding internal TATA-boxes, chi-sites and ribosomal entry sites; AT-rich or GC-rich sequence stretches; ARE, INS, CRS sequence elements; repeat sequences and RNA secondary structures; splice donor and acceptor sites, branch points. The term "vector" as used herein refers to a vehicle for transferring genetic material into a cell, wherein plasmids, viral vectors, cloning vectors, expression vectors, transcription vectors, artificial particles and artificial chromosomes are included. The vector comprises double or single stranded nucleic acids as DNA or RNA and includes at least a transgene, a backbone and optionally a promoter and a marker. Preferably the vector comprises a sequence comprising a sequence encoding a promoter of a green fluorescent protein, a green fluorescent protein itself, a promoter of preferably a cytomegalie virus, a gag gene, a pol gene and a sequence comprising long terminal repeats, which comprises preferably a deletion. The preferred vector includes amongst others PFV, MLV, HIV-1, bovine foamy viral vector (BFV), equine foamy viral vector (EFV), feline foamy viral vector (FFV), SFV chimpanzee (cpz), SFV macaque (mac), SFV African green monkey (agm), SFV orangutan (ora), SFV spider monkey (spm), SFVpcz, e.g. pczHFV, pczHSRV2 and pczDL; pci, e.g. pciSFV; pMH, e.g. pMH71, pMH118, pMH120; pMD, e.g. pMD9, pMD11; pCL1; pCAMSdeltaU3E and a nucleic acid comprising an MGMT gene with a point mutation p140k. The term "infectivity" as used herein refers to an entry of a vector into a host cell. Thereby, a nucleic acid is introduced into the host cell, and the host's and particle's membranes can fuse. The term "infectivity" also includes penetration, transduction, transfection and transformation. Infectivity can be enhanced by certain procedures or reagents e.g. to penetrate the cell membrane or to promote fusion or penetration. The transferred genetic material can be expressed or inserted, and genetic recombination can occur. Infectivity is measured e.g. by FACS analysis or progenitor assays indicated as relative infectivity normalized to VSV-G or absolute infectivity quoted as EGFP ffu/ml. Infection can be achieved by different methods, like by means of calcium phosphate, polyethyleneimine (PEI) or non-liposomale lipid agents, like PolyFect ® (Qiagene) and Fugene ® HD (Roche Diagnostics, Basel, Switzerland).

Genes foreign to a species are not expressed with optimal efficiency in hosts cells, since the codons in the foreign genes do not reflect the typical codon usage of the host organism. Therefore, to improve expression levels of env, their env codon was optimized by a specific exchange of certain codons. Hereafter is an exemplary selection of codons that were exchanged by optimized codons according to the invention.

Table 1

| original codons | optimized codons |
|---|---|
| gca, gcg | Gcc |
| gaa, gta | Gag |
| aga, agg | Cgg |
| agg | Cgg |
| att | Atc |
| aca, act | Acc |
| aat | Aac |
| gta | Gtg |
| ata | Atc |
| agt | Agc |
| cat | Cac |
| ctt, ctc | Ctg |
| caa | Cag |
| cgc | Cgg |
| caa | cct, ccc |
| tta, ttg | Ctg |

The listed codons were not completely exchanged, i.e. at specific locations of the nucleic acid the non-optimized codons were exchanged and at other specific locations the codons remained unchanged. Therefore, a very specific selection of codons was exchanged at very specific locations of the nucleic acid to get an optimized expression of env and consequently an improved infectivity and transduction. For example gaa and agg codons were exchanged at specific locations of PE01, SE01 and SE03 and remained at certain locations unchanged. Thereby, codon usage was adapted to the bias of *Homo sapiens* resulting in a high codon adaptation index value of 0.98. Infectivity and transduction of viral vectors comprising codon-optimized nucleic acids according to the invention were improved up to about 50-fold compared to the original non-optimized nucleic acids (Figs. 7 and 8). In addition to the codon-optimization the expression-optimization of env includes skipping of negatively cis-acting motifs as splice sites and poly(A) signals, and the low GC content of wt env was increased to prolong mRNA lifetime. Internal TATA-boxes, chi-sites, RNA secondary structure and ribosomal entry sites were avoided.

In a preferred embodiment the expression-optimized nucleic acid comprising a foamy viral envelope gene encoding a foamy viral envelope polypeptide, the foamy viral envelope gene comprises at least one mutation, wherein the mutation leads on expression of the foamy viral envelope gene to a lysine exchange in the envelope polypeptide.

In another preferred embodiment lysine is exchanged against an amino acid selected from the group consisting of alanine, histidine, glycine and arginine.

PE01, PE02 and PE03 are expression-optimized sequences derived from EM140, EM167 and EM168, respectively. SE01, SE02 and SE03 are expression-optimized sequences derived from SM04, SM05 and SM06, respectively. Infectivities and transductions for viral vectors comprising PE01, PE02 or PE03 are improved up to about 50-fold, 10-fold and 6-fold compared to the original non-optimized nucleic acids EM140, EM167 and EM168, respectively.

The present invention is objected to a method for preparing at least one pseudotyped vector particle comprising the steps of:

a) providing at least one cell;

b) adding to the cell a non-foamy viral vector and a nucleic acid, which comprises a foamy viral envelope gene encoding a foamy viral envelope polypeptide, the foamy viral envelope gene comprises at least one mutation, which leads on expression of the mutated foamy viral envelope gene to a modified envelope polypeptide, which comprises at least one inactivated ubiquitination site; and

c) harvesting at least one pseudotyped vector particle produced by the cell.

The invention is also directed to a method for preparing at least one pseudotyped vector particle comprising the steps of:

d) providing at least one cell;

e) adding to the cell at least one viral vector and an expression-optimized nucleic acid comprising a foamy viral envelope gene encoding a foamy viral envelope polypeptide, the foamy viral envelope gene comprises at least one mutation, which leads on expression of the mutated foamy viral envelope gene to a modified envelope polypeptide, which comprises at least one inactivated ubiquitination site, the modified envelope polypeptide pseudotypes the viral vector to prepare the pseudotyped vector particle for infecting the host cell; the infectivity of the pseudotyped viral vector particle is increased by expression-optimization up to about 50-fold compared to a non-optimized nucleic acid encoding the same modified envelope polypeptide; and

f) harvesting at least one pseudotyped vector particle produced by the cell.

The term "pseudotyping" and "pseudotyped" as used herein refers to a process or status, when a viral envelope polypeptide that is endogenous to the vector particle is replaced by a foreign polypeptide. The foreign polypeptide is derived from a virus that differs from the pseudotyped virus at least like two viruses from different species. The foreign polypeptide can also be a chimeric polypeptide. The term "cell" as used herein comprises in vivo systems, like single cells or multicellular organisms; ex vivo systems, like explants and slice cultures; and in vitro system, like cell culture systems. In a preferred embodiment of the invention the cell comprises a mammalian, like mice, rats, guinea pigs, pigs, marmosets and primates; HEK cells, like a HEK293T cell; fibrosarcoma cells, like HT1080; and hematopoietic cells, like CD34+ cells, HEL, HL60 and K562. As can be seen in Figs. 20, 21 and 23 hematopoietic cells, especially CD34+ cells, HEL, HL60 and K562 are very efficiently infected. The term "vector particle" as used herein refers to a vehicle of genetic information that is packed by substances including among others membranes, envelope polypeptides, capsid polypeptides and other glycoproteins. Optionally enzymes, like polymerases can also be packed within a vector particle. The term "vector particle" includes also viral particles and viral subparticles. The vector particle can be functional or non-functional. The term "adding" as used herein refers to any kind of bringing a vector into contact with a cell. The term "adding" includes among others mixing, blending, shaking, overlaying, applying, administering, putting together, loading, multiple loadings, incubating, culturing and pipetting. The term "harvesting" as used herein refers to any kind of collecting and processing of viral vectors from an intracellular and/or extracellular space, e.g. a supernatant of a cell culture. The term "harvesting" includes among others lysis of cells as well as sedimentation, centrifugation, filtration, concentration, extraction, purification and induction of vector particles. For preparing pseudotyped vector particles the polypeptide synthesis system of the host cell was used. The viral vector comprised endogenous nucleic acids, e.g. gag and pol, and foreign nucleic acids encoding envelope polypeptides, like env, for pseudotyping. The cell took up the added viral vector, optionally integrated it into its genome, transcribed it and translated the transcribed RNA into viral polypeptides. Subsequently, the polypeptides were processed, and the vector particles including nucleic acids were assembled and released.

Vectors pseudotyped by the modified env according to the invention are highly efficient in gene transfer. Yield, ability to be concentrated, infectivity and stability are greatly increased by pseudotyping with the modified env according to the invention. Figs. 17 to 19 show the result of a preparation of pseudotyped vector particles according to the invention with different expression constructs, like CD/NL-BH or pcziGag/pcziPol. Viral titers are indicated as transfection units (TE) per ml calculated by the following formula:

$$TE / ml = \frac{\text{number of cells at the time of infection}}{100\%} \times \text{number of GFPpositive cells} \times \text{dilution factor}$$

The gained vector particles were concentrated by centrifugation, whereby a concentration up to 20-fold was achieved using e.g. env EM140. The yield of vector particles pseudotyped by env EM140 was about 8-times higher before and about 1.4-times higher after centrifugation compared to VSV-G in case the same lentiviral vector and expression construct were used. Therefore, the preparation of pseudotyped vector particles according to the invention results in a higher yield and a more effective concentration of pseudotyped vector particles. The stability of gene transfer or the stability of expression of transferred genes attained by pseudotyped vectors prepared according to the invention is demonstrated in Fig. 22. If vector particles pseudotyped by env EM140 were used the stability decreased to a significant lower degree within four further days of cell culture compared to VSV-G provided that the same vector and expression construct were employed. In the case a prototype foamy viral vector was used instead of a lentiviral vector the expression of transferred genes even increase slightly.

In a preferred embodiment of the invention the method for preparing at least one pseudotyped vector particle comprises further the step of d) adding the harvested pseudotyped vector particle to at least one second cell, wherein efficiency of infecting the second cell by the vector particle pseudotyped by the modified envelope polypeptide is increased compared to a vector particle pseudotyped by a wild-type envelope polypeptide.

In another preferred embodiment of the invention the method further comprises the step of d) adding the harvested pseudotyped vector particle to at least one second cell, wherein efficiency of infecting the second cell by the vector particle pseudotyped by the modified envelope polypeptide is increased compared to a vector particle pseudotyped by an envelope polypeptide encoded by a non-optimized nucleic acid. The increased infectivity of pseudotyped viral vectors prepared according to the invention is demonstrated in Figs. 20 and 21 measured by FACS and progenitor assay, respectively. If a foamy viral envelope polypeptide is used to pseudotype a lentiviral vector the number of GFP positive cells is increased about almost 20% compared to the same lentiviral vector pseudotyped by VSV-G, and it is slightly increased compared to a prototype foamy viral vector enveloped by a foamy viral envelope polypeptide (Fig. 20). Clonal cells that were infected by a lentiviral vector pseudotyped by VSV-G were not detected. In contrast to that, more than about 50% GFP positive colonies were counted after an infection by a lentiviral vector pseudotyped by a foamy viral envelope polypeptide, e.g. env EM140. Thereby, the broad foamy viral host spectrum can be used in combination with non-foamy viral vectors. The pseudotyped vector particle is added to the second cell, whereby it transfers genetic material into the second cell, like MGMTP140K for therapeutic purposes (Figs. 18 and 24). The step of adding the pseudotyped viral particle to a second cell includes transduction, transfection or infection.

In another preferred embodiment of the invention the foamy viral envelope polypeptide is labeled by a marker. The term "marker" as used herein refers to a molecular label that can be a peptide or a non-peptide label linked to a molecule to be tracked. The marker can be detectable by e.g. colorimetric, fluorescent, spectroscopic or radioactive signals as well as by a secondary detection system, like streptavidine/biotin, whereby the signals can be also amplified.

In another preferred embodiment of the invention the marker is a fluorescent protein. The fluorescent protein includes preferably among others green fluorescent proteins, like EGFP, blue fluorescent proteins, like EBFP, red fluorescent proteins, like, DsRed and mCherry, yellow fluorescent proteins, like EYFP, cyan fluorescent proteins, like ECFP, and orange fluorescent proteins, like mOrange.

In a further preferred embodiment of the invention the foamy viral envelope polypeptide is tracked by an imaging technique. The imaging technique includes among others confocal microscopy, multi photon microscopy, fluorescent microscopy, tomography methods, e.g. fluorescence enhanced optical tomography, macroscopy, spectroscopy, positron emission tomography, molecular imaging, fluorescence resonance energy transfer based methods and FACS. The labeled foamy viral envelope polypeptide itself as well as a vector particle enveloped by the labeled polypeptide or a cell expressing the labeled polypeptide can be tracked.

In a further preferred embodiment of the invention the method for preparing at least one pseudotyped vector particle further comprises the step of: e) adding serum to the cell. As shown in Fig. 17 adding serum to the cell and/or to the viral vector increases the viral titer significantly, whereby the preparation of vector particles according to the invention gets more efficient. In a preferred embodiment 10% to 20% serum, more preferably 10%, 15% or 20% serum are added.

In another preferred embodiment of the invention at least one growth factor, e.g. a cytokine, like SCT, TPO, CSF, G-CSF, TPO, IL6 and FH3-L, is added to the cell, or growth factors are added together with serum.

In a further preferred embodiment of the invention the cell is provided in coated cell culture dishes, preferably coated by CH296, BSA or TCD.

In another preferred embodiment of the invention the method for preparing the pseudotyped vector particle is a high throughput method.

In an also preferred embodiment the nucleic acid added to the cell comprises a foamy viral envelope gene comprising at least one mutation, wherein the mutation leads on expression of the foamy viral envelope gene to a lysine exchange in the envelope polypeptide.

In a further preferred embodiment of the invention lysine is exchanged against an amino acid selected from the group consisting of alanine, histidine, glycine and arginine.

In a particularly preferred embodiment the nucleic acid added to the cell comprises a foamy viral envelope gene comprising at least one mutation, wherein the mutated foamy viral envelope gene is selected from the group consisting of EM135 to EM139, SEQ ID NO: 1 to 20 and SEQ ID NO: 21.

In a likewise preferred embodiment of the invention the viral vector added to the cell is derived from the family of *retroviridae.*In another preferred embodiment of the invention the retroviridae is selected from the group consisting of lentiviruses, alpha retroviruses, beta retroviruses, gamma retroviruses, delta retroviruses, epsilon retroviruses and spumaretrovirinae.

In an also preferred embodiment of the invention the viral vector and the mutated foamy viral envelope gene are located on a single construct. The term "construct" as used herein refers to any kind of vehicle that can carry nucleic acids. The term "construct" includes plasmids, vectors, artificial chromosomes and artificial particles. The viral vector and the mutated foamy viral envelope gene can follow each other on the construct or can be located at separate places of the construct.

In an additional preferred embodiment of the invention at least one expression construct is added comprising at least one gene selected from the group consisting of a gag gene, a pol gene and an envelope gene. The term "expression construct" as used herein refers to an expression vector that encodes auxiliary peptides, like a retroviral group-specific antigen and/or a retroviral polymerase. These auxiliary peptides are essential or supporting for viral morphogenesis, release, infectivity or other replicative functions. In a preferred embodiment the expression construct includes among others pzc vectors, like pczDWP001, pcziGag, pcziPol and pczCFG2fEGN; p6 vectors, like p6NST1, p6iPol and p6iGag; CD/NL-BH and cmvgp1. The term "gag" as used herein refers to a retroviral group-specific antigen, which encodes the internal structural proteins of the capsid. During maturation non-foamy viral gag is processed in its subunits comprising a matrix, a capsid and a nucleocapsid. The term "pol" as used herein refers to enzymatic components of retroviruses, like reverse transcriptase, integrase and protease.

The invention is also directed to a method for treating a genetic disorder comprising administering to a subject a nucleic acid comprising a foamy viral envelope gene comprising at least one mutation, wherein the mutation leads on expression of the mutated foamy viral envelope gene to a modified envelope polypeptide, which comprises at least one inactivated ubiquitination site. The term "treating" as used herein also comprises curing, preventing and attenuating a disorder or a disease. The term "treating" according to the invention also includes genetic engineering, genetic manipulation and gene therapy. By the method according to the invention a faulty gene is replaced by a functional gene or in the case of an absent gene a functional or additional gene is provided. The added gene can express a therapeutic polypeptide or provide a favorable property, like a chemotherapeutic resistance by MGMTP140K (Fig. 24). This is achieved by administering to a subject a nucleic acid comprising a mutated foamy viral envelope gene according to the invention. Therefore, the body can make the correct e.g. enzyme, regulator or protein also encoded by the administered nucleic acid, and consequently the root cause of a disease can be eliminated. Gene therapy includes the insertion of a normal gene into a non-specific location within the genome to replace a non-functional gene. An abnormal gene could also be swapped for a normal gene by homologous recombination or an abnormal gene could be repaired by selective reverse mutation, which returns the gene to its normal function. In addition, the regulation of a particular gene could be altered, i.e. the degree to which a gene is turned on or off. The term "genetic disorder" as used herein refers to hereditable and acquired disorders of genetic or epigenetic origin, like hemophilia, Lesch-Nyhan syndrome, retinoblastoma, phenylketonuria and metabolic diseases in which a defective gene causes e.g. an enzyme to be either absent or ineffective in catalyzing a particular metabolic reaction effectively. The term "genetic disorder" also includes among others acquired diseases and virally induced diseases, e.g. diseases of viral origin, like human papillomavirus, hepatitis B and hepatitis C virus, Epstein-Barr virus, human T-lymphotropic virus; cancer; metabolic diseases and AIDS. The method of this invention for preventing or treating a genetic disorder includes also the introduction of an env gene into a cell, wherein the cell becomes resistant to superinfection by other retroviruses with the same receptor specificity, a phenomenon base on receptor interference. The term "administering" as used herein refers to any kind of applying a gene or a gene comprising a substance to a subject.

The invention relates in addition to a method for treating a genetic disorder comprising administering to a subject a polypeptide encoded by a foamy viral envelope gene comprising at least one mutation, wherein the mutation leads on expression of the mutated foamy viral envelope gene to a modified envelope polypeptide, which comprises at least one inactivated ubiquitination site. The polypeptide comprising a modified env can be used as a carrier to deliver polypeptides and other substances, like drugs, enzymes, lipids and steroids, with a therapeutic effect to the target cell or organism. The administered polypeptide according to the invention can e.g. deliver enclosed DOPA to the brain cells of a Parkinson patient or an enclosed chemotherapeutical agent to the erythrocytes of a malaria patient, whereas the broad host range and the apathogenity of the env is of favor.

The invention is also directed to the use of an nucleic acid for the manufacture of a drug for the treatment and/or prevention of a genetic disorder, virally induced disease or cancer, the nucleic acid is expression-optimized and comprises a foamy viral envelope gene, which comprises at least one mutation, which leads on expression of the mutated foamy viral envelope gene to a modified envelope polypeptide, which comprises at least one inactivated ubiquitination site in the leader peptide, the modified envelope polypeptide pseudotypes a viral vector for infecting at least one host cell, wherein the foamy viral envelope gene is selected from the group consisting of SEQ ID NO: 7 (PE01), SEQ ID NO: 8 (PE02), SEQ ID NO: 9 (PE03), SEQ ID NO: 10 (SE01), SEQ ID NO: 11 (SE02) and SEQ ID NO: 12 (SE03). The infectivity is increased by expression-optimization up to about 50-fold compared to a non-optimized nucleic acid encoding the same modified envelope polypeptide.

**Methods**

**1. Cell culture techniques**

**1. Cloning of pczHFVenv EM167 and EM168**

PczHFVenv EM140 was linearised by EcoRI/NheI, and a PCR fragment PCR-0 digested with the same enzyme was inserted. The resulting construct pczHFVenv EM167 was used as vector for preparing pczHFVenv EM168. The overlapping PCR fragment PCR1/2 prepared as indicated in table 1 below was digested by EcoRI/NheI and ligated with pczHFVenv EM167 / EcoRI/NheI.

**Table 2**

| Vector | Template | Primers | PCR product |
|---|---|---|---|
| pczHFVenv EM167 | EM140 | 826 + 2271 | PCR-0 |
| pczHFVenv EM168 | EM167 | 2272 + 406 | PCR-1 |
| | EM167 | 826 + 2273 | PCR-2 |
| | PCR-1 + PCR-2 | 826 + 406 | overlap PCR1/2 |

**2. Preparation of vector particles**

**2.1 Transfection by PEI**

For a production of transient vector particles all nucleic acids were introduced into cells by co-transfection. Thereto, adherent cells were cultured on cell culture dishes, overlaid with a 0.1 % gelatine solution and incubated for 30 min to 3 days. On the first day of transfection the gelatine solution was removed, and HEK cells were seeded in a concentration of about 6 x $10^6$ HEK cells per 10 ml medium (DMEM). On the second day DMEM was replaced by a transfection medium containing 5 to 15 μg/ml PEI, 5 to 15 μg/ml DNA, DMEM and optionally 10 % - 20 % fetal calf serum (FCS). On the third day the cells with serum were washed with DMEM. About 10 mM to 20 mM sodium butyrate dissolved in PBS can be added to the medium of serum treated and non-treated cells. It was replaced after 6 hours by DMEM. On the fourth day at the earliest of 19 hours the supernatant containing vector particles was harvested and filtrated to remove cells and fragments thereof. The vector particles were either used immediately or stored at -80°C or -20 °C after detecting the viral titer.

### 2.2 Transfection by calcium phosphate

2 x $10^6$ cells per 5 ml medium, preferably DMEM, were incubated over night. On the following day 1/5 of the medium was removed, and a solution containing 423 μl distillated water, 62 μl of 2 M $CaCl_2$, 500 μl 2 x HBS and 15 μg DNA obtained by plasmid preparation was added (ratio solution/medium 1:5) to the cells. The DNA was applied to the cells as a precipitate of calcium phosphate/DNA.

2x HBS comprises:

| | |
|---|---|
| 50 mM | HEPES |
| 10 mM | KCl |
| 12 mM | dextrose x $H_2O$ (glucose monophosphate) |
| 280 mM | NaCl |
| 1.5 mM | $Na_2HPO4$ x 2 $H_2O$ |

After 7 hours of further incubation the medium was changed, and one day after transfection the cells were treated by sodium butyrate as described above (see 2.1).

### 2.3 Transfection by polyfect

1.6 x $10^6$ cells per 5 ml medium, preferably DMEM, were incubated over night. On the following day the medium was replaced by pre-heated medium (4ml per well). 6μ g DNA and 30 μl polyfect were mixed in 114 μl medium, incubated for 15 min at RT, and subsequently 1 ml medium was added before the mixture was dropped to the cells. The cells were incubated overnight and subsequently treated by sodium butyrate as described above (see 2.1).

## 3. Preparation of vector particles

### 3.1 Preparation from supernatants

Supernatants from a cell culture containing vector particles were purified from debris by a short period of centrifugation (about 5 min at 1,200 rpm) and subsequently sterile filtrated. Obtained vector particles were pooled and centrifuged at 4°C for 2-3 hours (at about 25,000 rpm) by means of a dense gradient with 20% sucrose. Subsequently, the supernatant was drawn off, and invisible viral sediment was resuspended in fresh medium. Before transduction the viral titer was functionally detected.

### 3.2 Preparation of intracellular viral vectors

Cells were broken by freezing and thawing, and viral vectors were released. Thereto, washed transfected cells were placed on dry ice or kept at about -80°C until the medium was completely frozen. Subsequently, they were thawed at temperatures between RT and 37°C. Cell debris can be removed by filtration.

## 4. Transduction of hematopoietic and fibrosarcoma cells

Untreated multiple well plates were coated with a recombinant fibronectin fragment (e.g. CH296). After washing dishes the centrifuged viral sediment was diluted in 3 ml medium, distributed to 6 wells and incubated. After half an hour 100,000 cells of lines HT1080, K562, HL60 and HEL contained in 50 to 100 μl medium were pipetted to the vector particles and incubated. About 16 hours later fresh medium was added to the cells, and after further two days the cells were transferred to a flask to continue cell culturing. A minor part of the cells was analyzed by FACS in a solution of PBS with 1 % FCS and 1 μg propidium iodide to evaluate transfection efficiency by detecting the ratio of GFP positive cells to GFP negative cells.

## 5. Transduction of human CD34+ cells

The transduction of human CD34+ cells was performed on multiple well plates coated with a recombinant fibronectin fragment. The fibronectin was preloaded once or twice with fresh or thawed vector, and 15,000 to 20,000 human CD34+ cells per well were pipetted to the vector particles. In addition, cytokines SCT, TPO and G-CSF were added at a concentration of 100 ng/ml. Soonest 16 hours after transduction, cells were transferred to fresh medium and cultivated as described above.

### 6. Transduction of marmoset CD34+ cells

Transduction of marmoset cells was performed on multiple well plates coated with a recombinant fibronectin fragment. Thawed or fresh vector particles were centrifuged, diluted in 1 ml medium and then put to the coated wells. After half an hour CD34+ cells of marmosets were added and incubated with cytokines CSF, TPO, IL6 and FH3-L at a concentration of 100 ng/ml. The concentration of applied cells ranged from 10,000 to 60,000 cells per well. Soonest 16 hours after transduction, marmoset cells were transferred to fresh medium and cultivated as described above.

### 7. Progenitor assay

To detect clonal cells by a progenitor assay CD34+ cells were seeded at low concentrations (e.g. 200 to 500 cells per ml) on a viscous medium containing cytokines on the day after transduction. Differentiated cells die during culture within a short period. Therefore, colonies generated by clonal cells were counted after 14 days of incubation under a fluorescence microscope, and the ratio of GFP positive and GFP negative cells was detected.

### 8. FACS analysis

A GFP gene was used as reporter gene. To count transfected cells expressed GFP was detected by its excitation by a laser beam. Thereto, cells were incubated with trypsin-EDTA for 5 to 10 min. The trypsin treatment was stopped by medium, and the cell suspension was centrifuged and immediately measured via FACS.

Alternative β-galactosidase staining was performed, and lacZ was used as a reporter gene

### 9. Tracking experiments

To detect vector particles coupled to a labeled env gene confocal laser scanning microscopy was used. Filter systems and laser types were chosen to detect GFP (absorption: 498 nm, emission: 516 nm) and dsRed (absorption: 556 nm, emission: 583 nm) or mCherry (absorption: 587, emission: 610) within a sample in vitro or in vivo. For in vitro tracking experiments cells e.g. HT1080 were grown on coated cover slips. After transfection they were washed with PBS and optionally fixed by paraformaldehyde (3%) at RT for 15 to 30 min or observed in vivo. After three more washing steps with PBS/glycine fixed or unfixed cells were observed by a confocal laser scanning microscope. For in vivo tracking experiments with low resolution a macroscopic fluorescent system (Leica MacroFluo™) is used for a non-invasive observation of labeled env in large samples, like behaving mice. For a higher resolution multi-photon microscopy is used to detect labeled env in vivo or ex vivo, e.g. in anesthetized animals or in tissue slices.

## Results

### 1. Preparation of vector particles

Figs. 17-19 show the results of pseudotyping foamy viral and orthoretroviral vectors with mutated env according to the invention derived from a foamy virus. Enveloping lentiviral, foamy viral and gamma retroviral vectors comprising env EM140 resulted in an increased viral titer compared to VSV-G. The gained vector particles were concentrated by centrifugation, whereby a concentration up to 20-fold was achieved using env EM140. The yield of vector particles pseudotyped by env EM140 was about 8-times higher before and about 1.4-times higher after centrifugation compared to VSV-G in case the same lentiviral vector pCL1 and expression construct CD/NL-BH were used. The yield of env EM140 pseudotyping pCL1 / CD/NL-BH was about $3.8x10^7$ TE/ml and was concentrated 2-fold. VSV-G pseudotyping pCL1 achieved only a yield of about 0.5 $x10^7$ TE/ml, and env EM 140 enveloping pCL1 produced only 0.8 $x10^7$ TE/ml before centrifugation. Env EM140 pseudotyping pCAMΔU3E provided the highest concentration of 5.5-fold by centrifugation. Therefore, the preparation of env EM140 pseudotyped vector particles resulted in a higher yield and a more effective concentration of pseudotyped vector particles. To further improve transfection efficiency serum was added to the transfection mixture. As shown in Fig. 17, in all cases yield of vector particles was increased by the serum. This serum increase was more than 6-fold for env EM140 pseudotyping pCL1, but only 2.5 for VSV-G pseudotyping pCL1. Therefore, the addition of serum increased the vector particle titer. This effect was enhanced by env EM140.

### 2. Gene transfer to CD34+ cells

The portions of GFP positive cells reflecting gene transfer rate into CD34+ cells is shown in Figs. 20 and 21 for human CD34+ cells and in Fig. 22 for marmoset CD34+ cells. PCL1 and MH71 enveloped by EM140 had the highest gene transfer rate with about 77 % and about 74 % GFP positive human cells, respectively, measured by FACS. For comparison a lentiviral vector was pseudotyped by VSV-G, which showed a gene transfer rate that was almost 20% lower compared to env EM140 / pCL1. If the same experiments were performed by a progenitor assay clonal cells comprising transferred genes were evaluated, wherein clonal cells are the more important cell type for gene therapy. Pseudotyping pCL1 by env EM140 achieved more than 50-fold increase of gene transfer compared to VSV-G showing no GFP positive clonal cells. Therefore, the transduction efficiency was clearly increased by enveloping foamy viral and orthoretroviral vectors with env EM140. Fig. 22 shows the gene transfer rate in marmoset CD34+ cells sorted by FACS on day 5 and day 9 after transduction. Compared to pCL1 pseudotyped by VSV-G the number of GFP positive cells was almost 10-fold higher for pCL1 pseudotyped by env EM140. From day 5 to day 9 after transduction the portion of GFP positive cells slightly increased (about 1%) for MH71 enveloped by env EM140, and it decreased only about 2% for pCL1/env EM140. However, the number of cells labeled by pCL1/VSV-G decreased clearly of about 60% within four days. Therefore, the transduction stability was also improved by env EM140 for marmoset CD34+ cells.

### 3. Transduction of hematopoietic cell lines

Cells of lines HL60, HEL and K562 were transduced with the vectors MH71, MH71.MGMT, MD9 and MD9.MGMT using EM140. For the vectors containing MD9 the expression construct pcziGag and pcziPol were added. The centrifuged viral sediment was resuspended in 3 ml medium with a titer of 1.7 x 106 TE/ml (MH71.MGMT), 1.4 x 107 TE/ml (MD9.MGMT), 1.8 x 107 TE/ml (MD9) and 2.5 x 107 TE/ml (MH71). Figure 23 shows that the portion of GFP positive cells depends on the used cell line, wherein HEL cells display the most efficient transduction. With HL60 cells the portion of GFP positive cells remained constant during a long period of time, e.g. 8 weeks. Therefore, HL60 and HEL cells are preferred for transduction.

### 4. MGMTp140k and resistance against BCNU

In addition, the resistance of transduced cells comprising the transgene MGMTp140k against BCNU was tested. Fig. 24 shows the percentage of GFP positive cells of different cell lines that survive a treatment with 0, 20, 80 and 160 μM of the cytostatic drug BCNU. The amount of GFP positive cells based on the total amount of surviving cells is increased by the transgene MGMTp140k, which provides resistance against BCNU. Therefore, it was shown that genes like MGMTp140k can be successfully transduced.

### 5. Infectivity of pseudotyped vector particles

#### 5.1 Env EM167, EM168, EM225, EM226, SM05 and SM04

The modified env according to the invention, which comprises at least one inactivated ubiquitination site located at TM of the modified envelope polypeptide were used to pseudotype different types of viruses. The absolute infectivities and relative infectivities of these env pseudotyped viruses applied to eukaryotic cells were measured. The relative infectivity was normalized to the same viruses pseudotyped by VSV-G. As a result, there was a clear increase of infectivity due to the at least one inactivated ubiquitination site located at TM of the modified envelope polypeptide. As can be seen in Figs. 5 and 6 the infectivities rose about 2-times by env EM225 and env EM226 compared to env EM002 for pseudotyped HIV-1 vectors and MLV vectors. Env EM167 and env EM168 enhanced infectivities about 3-times compared to env EM140 for pseudotyped HIV-1 vectors. The infectivities were increased about 3-times and 4-times by env SM05 and env SM06, respectively, compared to env SM04 for pseudotyped HIV-1 vectors, and about 2- times and 5-times, respectively, for pseudotyped MLV vectors. Therefore, the efficiency of viral transduction and gene transfer was raised by the modified env EM167, EM168, EM225, EM226, SM05 and SM06.

#### 5.2 Env PE01, PE02 and PE03

Relative and absolute infectivities of viral vectors enveloped or pseudotyped by the modified env encoded by codon optimized env genes according to the invention were highly improved (Figs. 7, 8). Using PE01 infectivity rose about 11-times for pseudotyped HIV-1 vectors and 49-times for pseudotyped MLV vectors compared to env EM140. The infectivities were increased about 9-times by PE02 for pseudotyped HIV-1 vectors and 6-times for pseudotyped MLV vectors compared to env EM167. PE03 enhanced infectivity 6-times for pseudotyped MLV and HIV-1 vectors compared to env EM168. Therefore, the efficiency of viral transduction and gene transfer was raised by expression-optimized env genes PE01 - PE03.

### 5.3 Env EM228, env EM170 and env EM171

Relative and absolute infectivities of viral vectors enveloped or pseudotyped by the modified env comprising deletions according to the invention were also highly improved (Figs. 10, 11). Using env EM228 infectivity rose about 13-times for pseudotyped HIV-1 vectors and 82-times for pseudotyped MLV vectors compared to env EM042. The infectivities were increased about 3-times by env EM170 for pseudotyped HIV-1 vectors and MLV vectors compared to env EM043. Env EM171 enhanced infectivity 6-times for pseudotyped MLV and HIV-1 vectors compared to env EM070. Therefore, the efficiency of viral transduction and gene transfer was raised by modified env EM228, EM170, EM171.

### 6. Tracking env

Cells transfected with vector particles comprising env EM238, env EM236 or env EM261 show fluorescent signals in the cytoplasm (predominantly around the nucleus) and in the cell membrane. Vector particles enveloped or pseudotyped by env EM238, env EM236 or env EM261 can be also detected as small fluorescent dots with a size of about 100 nm.

### 7. Sequence listing

| SEQ ID NO: | Internal reference: |
|---|---|
| 7 | PE01 |
| 8 | PE02 |
| 9 | PE03 |
| 10 | SE01 |
| 11 | SE02 |
| 12 | SE03 |

**References:**

Anderson et al., Hum. Gene. Ther. 1(3), pp. 331-62, 1990
Check, Nature 433, p. 561, 2005
Nienhuis et al., Ann. N. Y. Acad. Sci. 996, pp.101-11, 2003
Li et al., J. Hum. Virol. 1(5), pp. 346-52, 1998
Li et al., Science 296, p. 497, 2002
Lindemann et al., J. Virol. 75, pp. 5762-5771, 2001
Russel and Miller, J. Virol. 70, pp. 217-222, 1996
Stanke et al., J. Virol. 79, pp. 15074-15083, 2005
Schmidt and Rethwilm, Virology 210, pp. 167-178, 1995
Weiss, Nature 380, p. 201, 1996
Wilk et al., J. Virol. 74, pp. 2885-2887, 2001
US 6,111,087
US 5,929,222

## Claims

**1.** A nucleic acid comprising a foamy viral envelope gene encoding a modified foamy viral envelope polypeptide, wherein

- the nucleic acid is expression-optimized,
- the foamy viral envelope gene comprises at least one mutation, which leads on expression of the mutated foamy viral envelope gene to a modified envelope polypeptide, and
- the modified envelope polypeptide comprises at least one inactivated ubiquitination site in the leader peptide, and pseudotypes a viral vector for infecting at least one host cell,

wherein
the foamy viral envelope gene is selected from the group consisting of SEQ ID NO: 7 (PE01), SEQ ID NO: 8 (PE02), SEQ ID NO: 9 (PE03), SEQ ID NO: 10 (SE01), SEQ ID NO: 11 (SE02) and SEQ ID NO: 12 (SE03).

**2.** A method for preparing at least one pseudotyped vector particle comprising the steps of:

a) providing at least one cell;
b) adding to the cell at least one viral vector and a nucleic acid of claim 1; and
c) harvesting at least one pseudotyped vector particle produced by the cell.

**3.** The method of claim 2, further comprising the step of:

d) adding the harvested pseudotyped vector particle to at least one second cell,

wherein efficiency of infecting the second cell by the vector particle pseudotyped by the modified envelope polypeptide is increased compared to a vector particle pseudotyped by an envelope polypeptide encoded by a non-optimized nucleic acid.

4. The method of claim 2 or 3, wherein the foamy viral envelope polypeptide is labeled by a marker.

5. The method of claim 4, wherein the labeled foamy viral envelope polypeptide is tracked by an imaging technique.

6. The method of any of claims 2 to 5, further comprising the step of:

   e) adding serum to the cell.

7. The method of any of claims 2 to 6, wherein the viral vector is derived from the family of retroviridae.

8. The method of claim 7, wherein the retroviridae is selected from the group consisting of lentiviruses, alpha retroviruses, beta retroviruses, gamma retroviruses, delta retroviruses, epsilon retroviruses and spumaretrovirinae.

9. The method of any of claims 2 to 8, wherein the viral vector and the mutated foamy viral envelope gene are located on a single construct.

10. The method of any of claims 2 to 9, wherein at least one expression construct is added comprising at least one gene selected from the group consisting of a gag gene, a pol gene and an envelope gene.

11. A nucleic acid of claim 1 for the treatment and/or prevention of a genetic disorder, a virally induced disease and/or cancer.

**Patentansprüche**

1. Nukleinsäure umfassend ein foamyvirales Hüllgen, das für ein foamyvirales Hüllpolypeptid codiert, wobei

   - die Nukleinsäure expressionsoptimiert ist,
   - das foamyvirale Hüllgen mindestens eine Mutation aufweist, die durch Expression des foamyviralen Hüllgens zu einem abgewandelten Hüllpolypeptid führt, und
   - das abgewandelte Hüllpolypeptid mindestens eine inaktivierte Ubiquitinylierungsstelle im Leader-Peptid aufweist, und einen viralen Vektor zum Infizieren von mindestens einer Wirtszelle pseudotypisiert,

   wobei das foamyvirale Hüllgen ausgewählt ist aus der Gruppe bestehend aus SEQ ID NO: 7 (PE01), SEQ ID NO: 8 (PE02), SEQ ID NO: 9 (PE03), SEQ ID NO: 10 (SE01), SEQ ID NO: 11 (SE02) and SEQ ID NO: 12 (SE03).

2. Verfahren zum Herstellen mindestens eines pseudotypisierten Vektorpartikels, umfassend die Schritte:

   a) Bereitstellen mindestens einer Zelle,
   b) Hinzufügen mindestens eines viralen Vektors und einer Nukleinsäure gemäß Anspruch 1 zu der Zelle,
   c) Ernten mindestens eines pseudotypisierten Vektorpartikels, der von der Zelle produziert wurde.

3. Verfahren gemäß Anspruch 2, weiterhin umfassend den Schritt:

   d) Hinzufügen des geernteten pseudotypisierten Vektorpartikels zu mindestens einer zweiten Zelle,

   wobei die Effizienz, mit der die zweite Zelle mit dem Vektorpartikel, der durch das abgewandelte Hüllpolypeptid pseudotypiesiert wurde, infizierte wird, erhöht ist im Vergleich zu einem Vektorpartikel, der durch ein Hüllpolypeptid pseudotypisiert wurde, das von einer nicht optimierten Nukleinsäure codiert wurde.

4. Verfahren gemäß Anspruch 2 oder 3, wobei das foamyvirale Hüllpolypeptid mit einem Marker gekennzeichnet ist.

5. Verfahren gemäß Anspruch 4, wobei das gekennzeichnete foamyvirale Hüllpolypeptid mittels eines bildgebenden Verfahrens verfolgt werden kann.

6. Verfahren gemäß einem der Ansprüche 2 bis 5, weiterhin umfassend den Schritt:

e) Hinzufügen von Serum zu der Zelle.

**7.** Verfahren gemäß einem der Ansprüche 2 bis 6, wobei der virale Vektor aus der Familie der Retroviren stammt.

**8.** Verfahren gemäß Anspruch 7, wobei die Retroviren ausgewählt sind aus der Gruppe bestehend aus Lentiviren, Alpha-Retroviren, Beta-Retroviren, Gamma-Retroviren, Delta-Retroviren, Epsilon-Retroviren und Spumaretroviren.

**9.** Verfahren gemäß einem der Ansprüche 2 bis 8, wobei der virale Vektor und das mutierte foramyvirale Hüllgen auf einem einzigen Konstrukt liegen.

**10.** Verfahren gemäß einem der Ansprüche 2 bis 9, wobei mindestens ein Expressionskonstrukt hinzugefügt wird, das mindestens ein Gen, ausgewählt aus der Gruppe bestehend aus gag-Gen, pol-Gen und Hüllgen, umfasst.

**11.** Nukleinsäure gemäß Anspruch 1 zur Behandlung und/oder Vorbeugung einer genetischen Störung, einer durch Viren verursachten Erkrankung und/oder Krebs.

**Revendications**

**1.** Un acide nucléique comprenant un gène d'enveloppe de spumavirus codant un polypeptide d'enveloppe de spumavirus modifié, dans lequel

- l'acide nucléique est optimisé pour expression
- le gène d'enveloppe de spumavirus comprend au moins une mutation, qui conduit à l'expression du gène d'enveloppe de spumavirus muté dans un polypeptide d'enveloppe modifié, et
- le polypeptide d'enveloppe modifié comprend au moins un site d'ubiquitination désactivé dans le peptide signal, et pseudotype un vecteur viral pour infecter au moins une cellule-hôte,

dans lequel
le gène d'enveloppe de spumavirus est sélectionné dans le groupe constitué par SEQ ID NO: 7 (PE01), SEQ ID NO: 8 (PE02), SEQ ID NO: 9 (PE03), SEQ ID NO: 10 (SE01), SEQ ID NO: 11 (SE02) et SEQ ID NO: 12 (SE03).

**2.** Un procédé pour préparer au moins une particule de vecteur pseudotypé comprenant les étapes suivantes:

a) fournir au moins une cellule;
b) ajouter au moins un vecteur viral et l'acide nucléique de la revendication 1 à la cellule, et
c) recueillir au moins une particule de vecteur pseudotypé produite par la cellule.

**3.** Procédé selon la revendication 2, comprenant en outre l'étape suivante:

d) ajouter la particule de vecteur pseudotypé recueillie à au moins une deuxième cellule,

dans lequel l'efficacité d'infection de la deuxième cellule par la particule de vecteur pseudotypé par le polypeptide d'enveloppe modifié est augmentée par rapport à une particule de vecteur pseudotypé par un polypeptide d'enveloppe codé par un acide nucléique qui n'a pas été optimisé.

**4.** Procédé selon la revendication 2 ou 3, dans lequel le polypeptide d'enveloppe de spumavirus est marqué par un marqueur.

**5.** Procédé selon la revendication 4, dans lequel le polypeptide d'enveloppe de spumavirus marqué est tracé par une technique d'imagerie.

**6.** Procédé selon l'une quelconque des revendications de 2 à 5, comprenant en outre l'étape suivante:

e) ajouter du sérum à la cellule.

**7.** Procédé selon l'une quelconque des revendications de 2 à 6, dans lequel le vecteur viral est dérivé de la famille *Retroviridae.*

8. Procédé selon la revendication 7, dans lequel la famille de *Retroviridae* est sélectionné dans le groupe constitué par *Lentivirus, Alpharetrovirus, Betaretrovirus, Gammaretrovirus, Deltaretrovirus, Epsilonretrovirus* et *Spumaretrovirinae.*

9. Procédé selon l'une quelconque des revendications de 2 à 8, dans lequel le vecteur viral et le gène d'enveloppe de spumavirus muté sont situés sur une seule construction.

10. Procédé selon l'une quelconque des revendications de 2 à 9, dans lequel au moins une construction d'expression est ajoutée, comprenant au moins un gène sélectionné dans le groupe constitué par un gène gag, un gène pol et un gène d'enveloppe.

11. Un acide nucléique selon la revendication 1 pour le traitement et/ou la prévention d'un trouble génétique, d'une maladie d'origine virale et/ou un cancer.

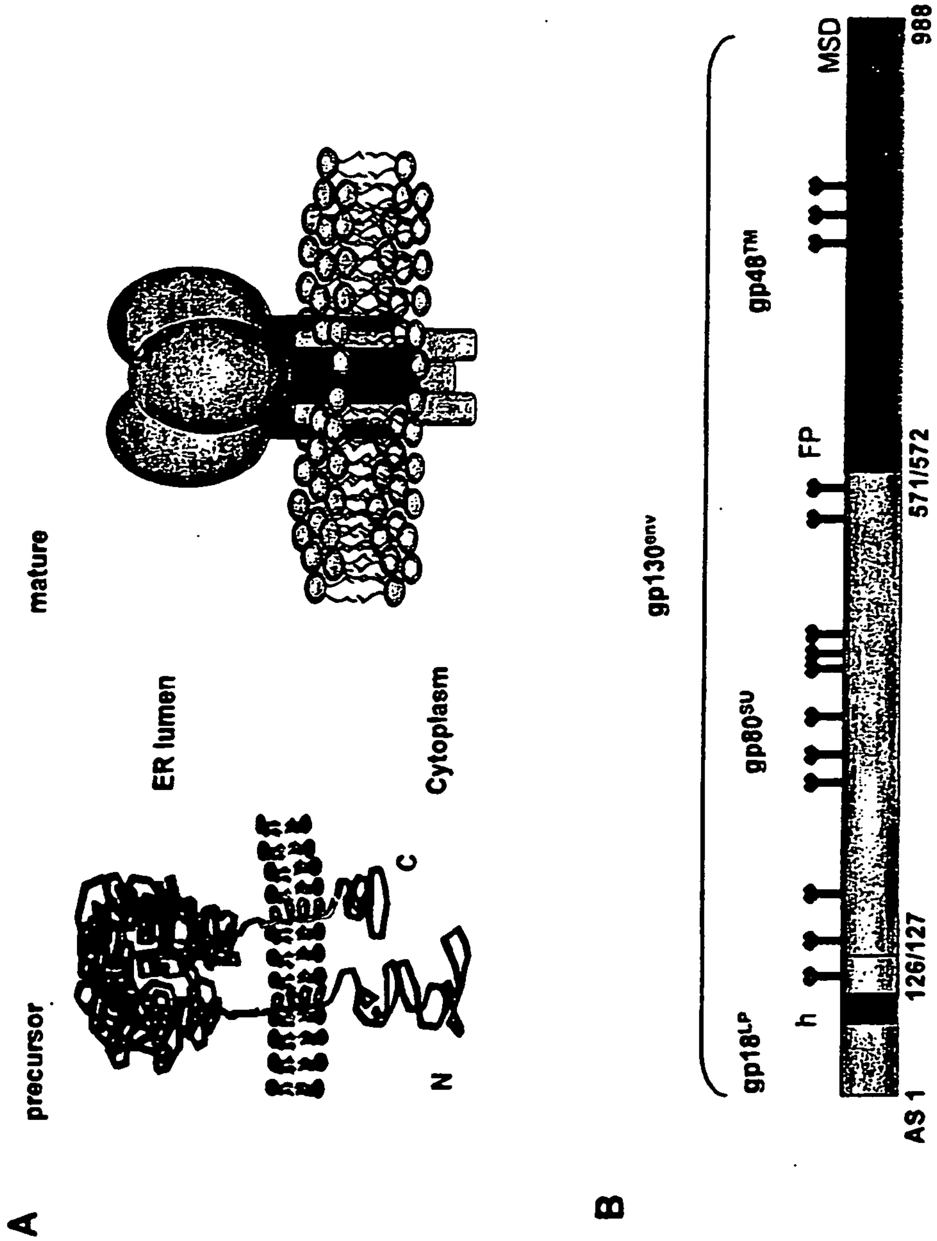
A
precursor
ER lumen
Cytoplasm
mature
N
C
B
gp130env
gp18LP
gp80SU
gp48TM
h
FP
MSD
AS 1
126/127
571/572
988

Fig. 1

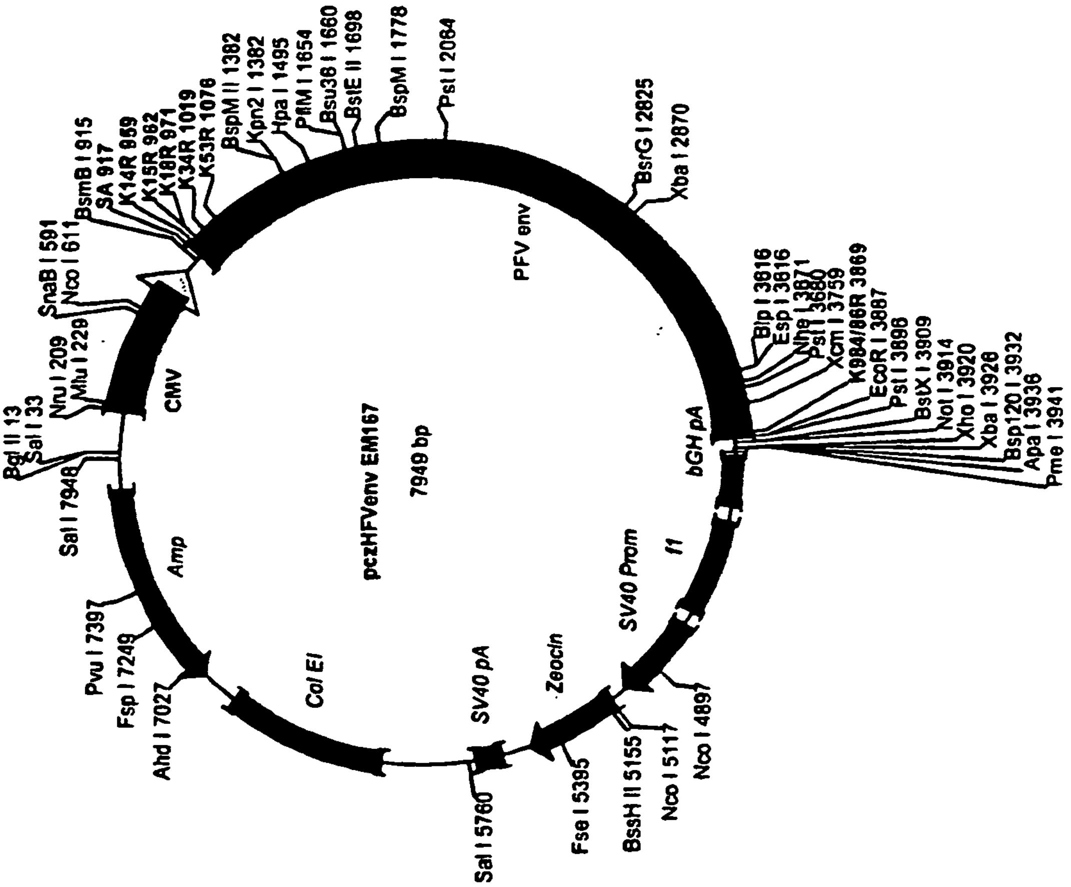
pczHFVenv EM167
7949 bp
CMV
PFV env
bGH pA
f1
SV40 Prom
Zeocin
SV40 pA
Col EI
Amp
Bgl II 13
Sal I 33
Nru I 209
Mlu I 229
SnaB I 591
Nco I 611
BsmB I 915
SA 917
K14R 959
K15R 962
K18R 971
K34R 1019
K53R 1076
BspM II 1382
Kpn2 I 1382
Hpa I 1495
PflM I 1654
Bsu36 I 1660
BstE II 1698
BspM I 1778
Pst I 2064
BsrG I 2825
Xba I 2870
Blp I 3616
Esp I 3616
Nhe I 3671
Pst I 3680
Xcm I 3759
K984/86R 3869
EcoR I 3887
Pst I 3896
BstX I 3909
Not I 3914
Xho I 3920
Xba I 3926
Bsp120 I 3932
Apa I 3936
Pme I 3941
Nco I 4897
Nco I 5117
BssH II 5155
Fse I 5395
Sal I 5760
Ahd I 7027
Fsp I 7249
Pvu I 7397
Sal I 7946

Fig. 2

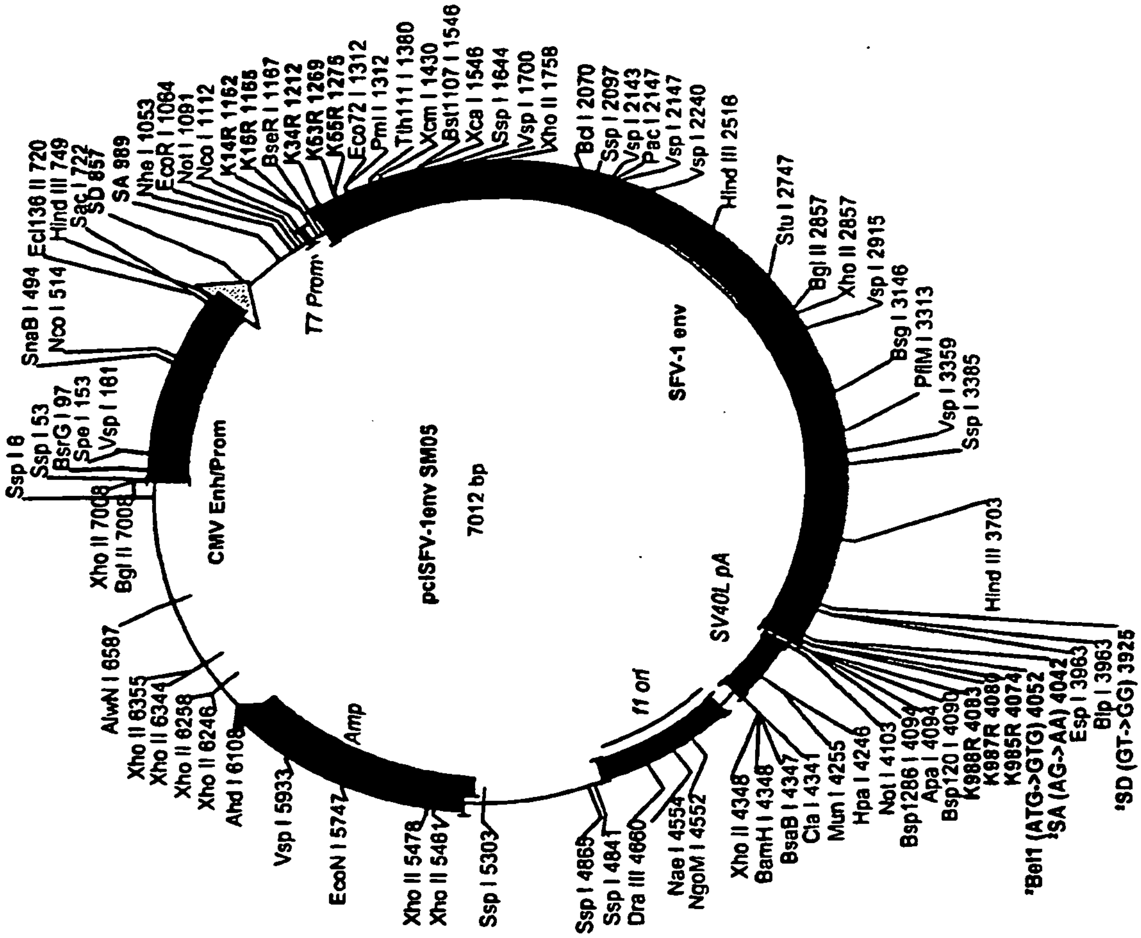
pciSFV-1env SM05
7012 bp
CMV Enh/Prom
T7 Prom
SFV-1 env
SV40L pA
f1 ori
Amp
Ssp I 8
Ssp I 53
BsrG I 97
Spe I 153
Vsp I 181
SnaB I 494
Nco I 514
Ecl136 II 720
Hind III 749
Sac I 722
SD 857
SA 989
Nhe I 1053
EcoR I 1064
Not I 1091
Nco I 1112
K14R 1162
K16R 1165
BseR I 1167
K34R 1212
K53R 1269
K55R 1275
Eco72 I 1312
Pml I 1312
Tth111 I 1380
Xcm I 1430
Bst1107 I 1546
Xca I 1546
Ssp I 1644
Vsp I 1700
Xho II 1758
Bcl I 2070
Ssp I 2097
Vsp I 2143
Pac I 2147
Vsp I 2147
Vsp I 2240
Hind III 2516
Stu I 2747
Bgl II 2857
Xho II 2857
Vsp I 2915
Bsg I 3146
PflM I 3313
Vsp I 3359
Ssp I 3385
Hind III 3703
'SD (GT->GG) 3925
Blp I 3963
Esp I 3963
'SA (AG->AA) 4042
'Bel1 (ATG->GTG) 4052
K985R 4074
K987R 4080
K988R 4083
Bsp120 I 4090
Apa I 4094
Bsp1286 I 4094
Not I 4103
Hpa I 4246
Mun I 4255
Cla I 4341
BsaB I 4347
BamH I 4348
Xho II 4348
NgoM I 4552
Nae I 4554
Dra III 4660
Ssp I 4841
Ssp I 4865
Ssp I 5303
Xho II 5461
Xho II 5478
EcoN I 5742
Vsp I 5933
Ahd I 6108
Xho II 6246
Xho II 6258
Xho II 6344
Xho II 6355
AlwN I 6587
Bgl II 7008
Xho II 7008

Fig. 3

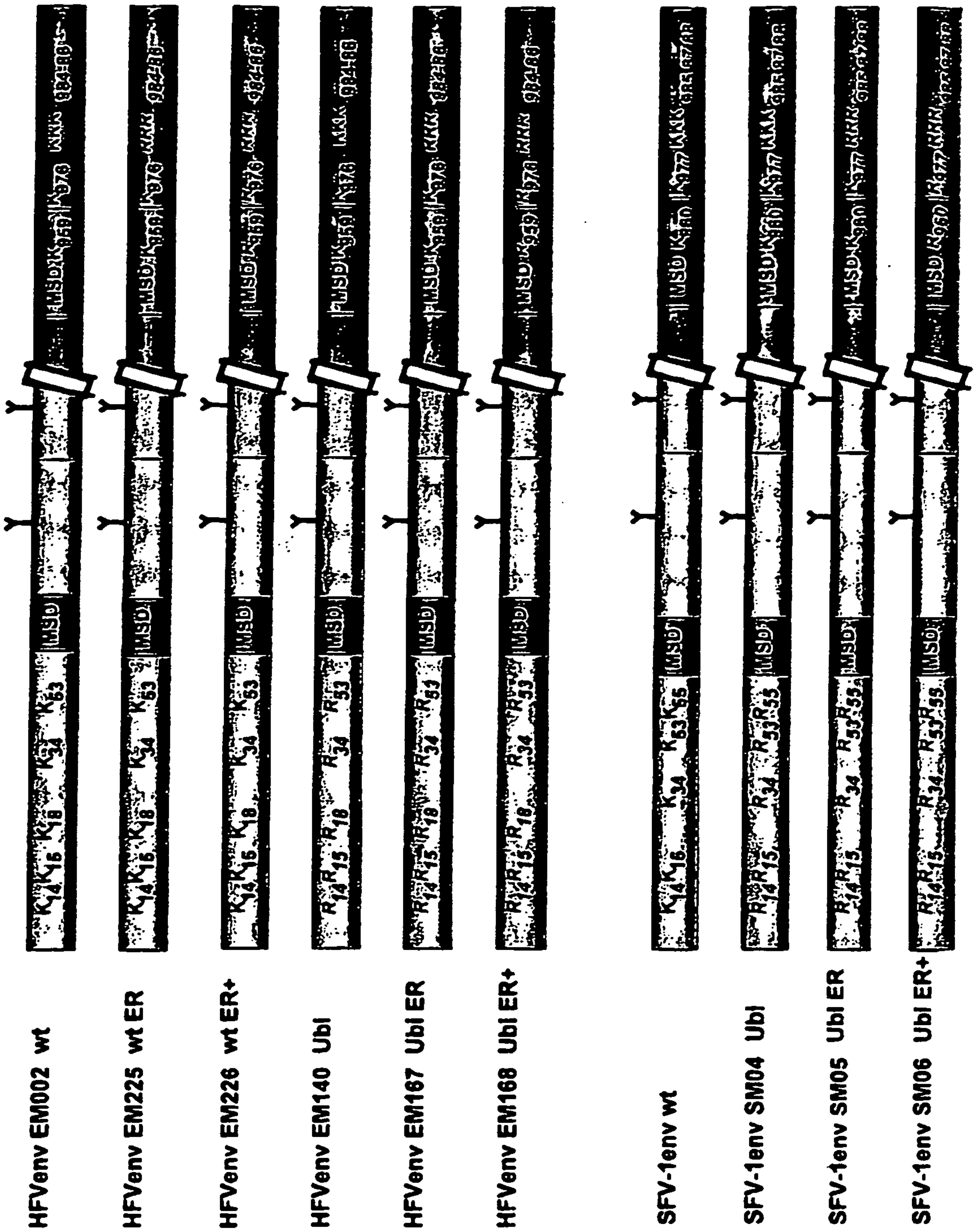
HFVenv EM002 wt
HFVenv EM225 wt ER
HFVenv EM226 wt ER+
HFVenv EM140 Ubl
HFVenv EM167 Ubl ER
HFVenv EM168 Ubl ER+
SFV-1env wt
SFV-1env SM04 Ubl
SFV-1env SM05 Ubl ER
SFV-1env SM06 Ubl ER+
MSD

Fig. 4

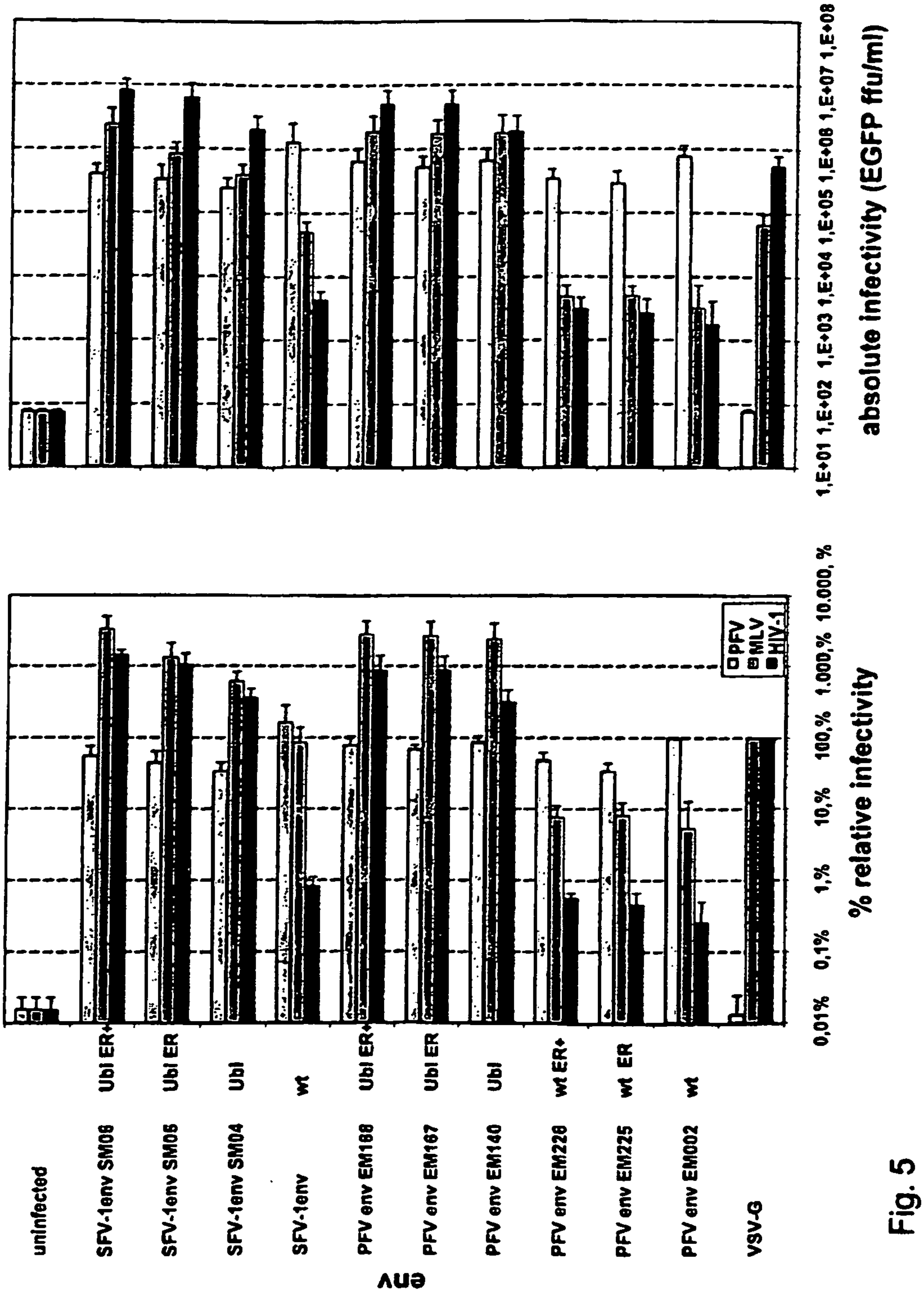
uninfected
SFV-1env SM08 Ubl ER+
SFV-1env SM06 Ubl ER
SFV-1env SM04 Ubl
SFV-1env wt
PFV env EM168 Ubl ER+
PFV env EM167 Ubl ER
PFV env EM140 Ubl
PFV env EM228 wt ER+
PFV env EM225 wt ER
PFV env EM002 wt
VSV-G
env
PFV
MLV
HIV-1
0,01% 0,1% 1,% 10,% 100,% 1.000,% 10.000,%
% relative infectivity
1,E+01 1,E+02 1,E+03 1,E+04 1,E+05 1,E+06 1,E+07 1,E+08
absolute infectivity (EGFP ffu/ml)

Fig. 5

| Vector System | Envelope env | relative Infectivity | | absolute Infectivity | |
|---|---|---|---|---|---|
| | | Mean | StdDev | Mean | StdDev |
| HIV-1 | VSV-G | 100,00% | 0,0% | 5,5E+05 | 2,4E+05 |
| | PFVwt, EM002 | 0,26% | 0,2% | 1,8E+03 | 2,3E+03 |
| | PFVwt ER, EM225 | 0,47% | 0,2% | 2,7E+03 | 1,7E+03 |
| | PFVwt ER+, EM226 | 0,57% | 0,1% | 3,2E+03 | 1,4E+03 |
| | PFV Ubi, EM140 | 325,24% | 137,0% | 1,9E+06 | 1,4E+06 |
| | PFV Ubi ER, EM167 | 905,25% | 488,2% | 5,2E+06 | 3,0E+06 |
| | PFV Ubi ER+, EM168 | 901,03% | 520,8% | 5,2E+06 | 3,1E+06 |
| | SFV-1wt, SM00 | 0,84% | 0,3% | 4,4E+03 | 1,5E+03 |
| | SFV-1 Ubi, SM04 | 369,34% | 119,9% | 2,1E+06 | 1,2E+06 |
| | SFV-1 Ubi ER, SM05 | 1067,78% | 451,8% | 6,4E+06 | 4,1E+06 |
| | SFV-1 Ubi ER+, SM06 | 1484,81% | 237,1% | 8,3E+06 | 3,8E+06 |
| MLV | VSV-G | 100,00% | 0,0% | 6,6E+04 | 2,8E+04 |
| | PFVwt, EM002 | 5,44% | 7,3% | 3,3E+03 | 4,0E+03 |
| | PFVwt ER, EM225 | 8,35% | 4,0% | 5,1E+03 | 1,9E+03 |
| | PFVwt ER+, EM226 | 7,82% | 3,1% | 5,0E+03 | 2,3E+03 |
| | PFV Ubi, EM140 | 2475,69% | 1618,3% | 1,8E+06 | 1,6E+06 |
| | PFV Ubi ER, EM167 | 2752,80% | 1506,1% | 1,8E+06 | 1,1E+06 |
| | PFV Ubi ER+, EM168 | 2861,47% | 1487,2% | 1,9E+06 | 1,4E+06 |
| | SFV-1wt, SM00 | 86,08% | 51,4% | 5,0E+04 | 2,2E+04 |
| | SFV-1 Ubi, SM04 | 629,41% | 219,2% | 4,0E+05 | 1,7E+05 |
| | SFV-1 Ubi ER, SM05 | 1359,37% | 791,6% | 8,4E+05 | 4,1E+05 |
| | SFV-1 Ubi ER+, SM06 | 3411,55% | 1680,0% | 2,4E+06 | 1,8E+06 |
| PFV | VSV-G | 0,01% | 0,0% | 7,6E+01 | 3,6E+00 |
| | PFVwt, EM002 | 100,00% | 0,0% | 7,9E+05 | 3,5E+05 |
| | PFVwt ER, EM225 | 34,07% | 9,6% | 2,9E+05 | 1,6E+05 |
| | PFVwt ER+, EM226 | 48,45% | 13,2% | 3,5E+05 | 1,3E+05 |
| | PFV Ubi, EM140 | 87,76% | 18,1% | 6,7E+05 | 3,2E+05 |
| | PFV Ubi ER, EM167 | 69,56% | 8,6% | 5,3E+05 | 2,3E+05 |
| | PFV Ubi ER+, EM168 | 78,37% | 24,4% | 6,6E+05 | 3,7E+05 |
| | SFV-1wt, SM00 | 166,16% | 116,2% | 1,3E+06 | 1,2E+06 |
| | SFV-1 Ubi, SM04 | 34,31% | 10,4% | 2,5E+05 | 1,0E+05 |
| | SFV-1 Ubi ER, SM05 | 45,33% | 18,5% | 3,4E+05 | 2,0E+05 |
| | SFV-1 Ubi ER+, SM06 | 56,65% | 19,2% | 4,0E+05 | 1,5E+05 |
| | uninfected | 0,02% | 0,0% | 7,6E+01 | 3,6E+00 |

Fig. 6

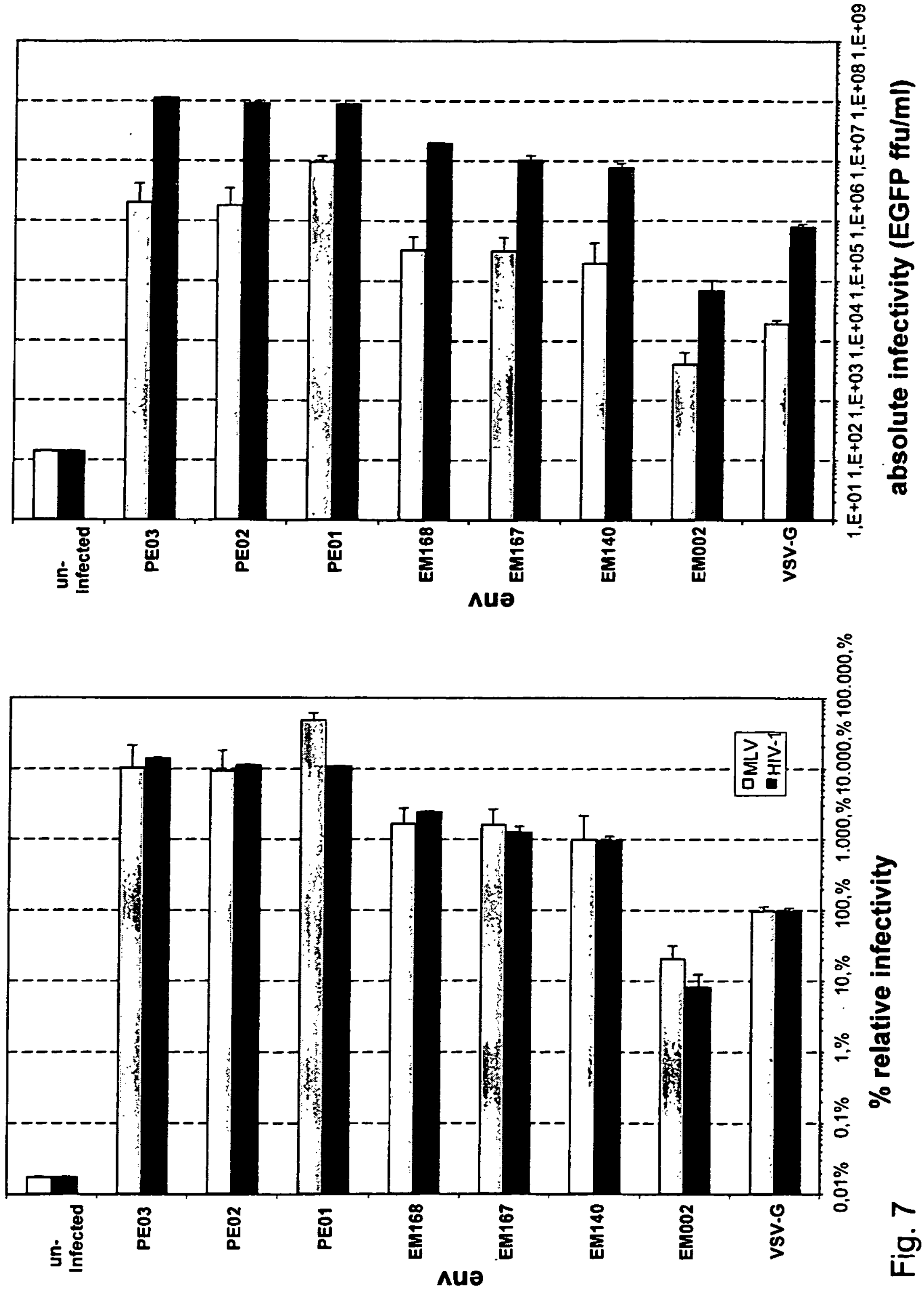
un-Infected
PE03
PE02
PE01
EM168
EM167
EM140
EM002
VSV-G
env
1,E+01 1,E+02 1,E+03 1,E+04 1,E+05 1,E+06 1,E+07 1,E+08 1,E+09
absolute infectivity (EGFP ffu/ml)
MLV
HIV-1
0,01% 0,1% 1,% 10,% 100,% 1.000,% 10.000,% 100.000,%
% relative infectivity

Fig. 7

| Vector System | Envelope env | relative infectivity | | absolute infectivity | |
|---|---|---|---|---|---|
| | | Mean | StdDev | Mean | StdDev |
| HIV-1 | VSV-G | 100,00% | 7,4% | 8,2E+05 | 6,1E+04 |
| | EM002 | 8,47% | 4,0% | 6,9E+04 | 3,3E+04 |
| | EM140 | 982,21% | 124,1% | 8,1E+06 | 1,0E+06 |
| | EM167 | 1295,86% | 226,9% | 1,1E+07 | 1,9E+06 |
| | EM168 | 2483,57% | | 2,0E+07 | |
| | PE01 | 11101,25% | | 9,1E+07 | |
| | PE02 | 11416,80% | | 9,4E+07 | |
| | PE03 | 14132,97% | | 1,2E+08 | |
| MLV | VSV-G | 100,00% | 12,8% | 2,0E+04 | 2,5E+03 |
| | EM002 | 20,94% | 10,9% | 4,1E+03 | 2,2E+03 |
| | EM140 | 1013,49% | 1157,2% | 2,0E+05 | 2,3E+05 |
| | EM167 | 1633,18% | 1054,1% | 3,2E+05 | 2,1E+05 |
| | EM168 | 1690,35% | 1078,1% | 3,3E+05 | 2,1E+05 |
| | PE01 | 49512,26% | 11465,7% | 9,8E+06 | 2,3E+06 |
| | PE02 | 9407,12% | 8549,7% | 1,9E+06 | 1,7E+06 |
| | PE03 | 10464,81% | 10706,9% | 2,1E+06 | 2,1E+06 |
| | uninfected | 0,02% | 0,0% | 1,4E+02 | 0,0E+00 |

Fig. 8

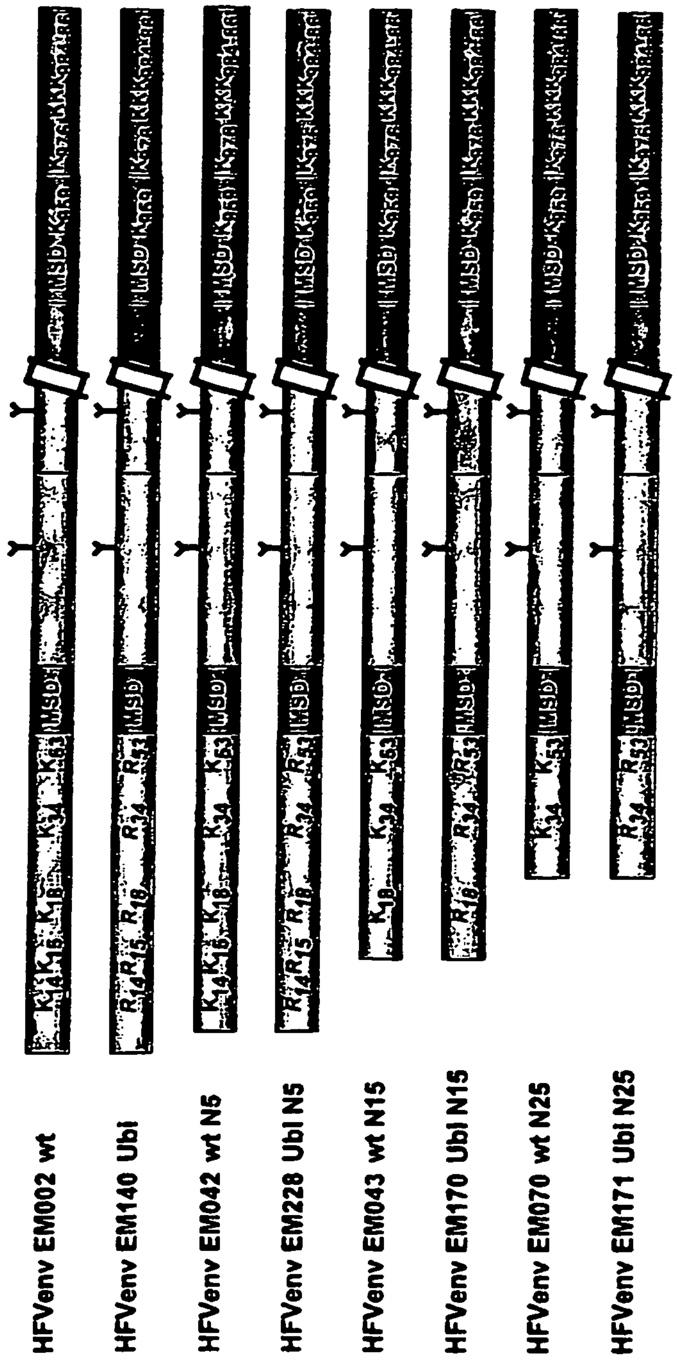
HFVenv EM002 wt
HFVenv EM140 Ubl
HFVenv EM042 wt N5
HFVenv EM228 Ubl N5
HFVenv EM043 wt N15
HFVenv EM170 Ubl N15
HFVenv EM070 wt N25
HFVenv EM171 Ubl N25
MSD

Fig. 9

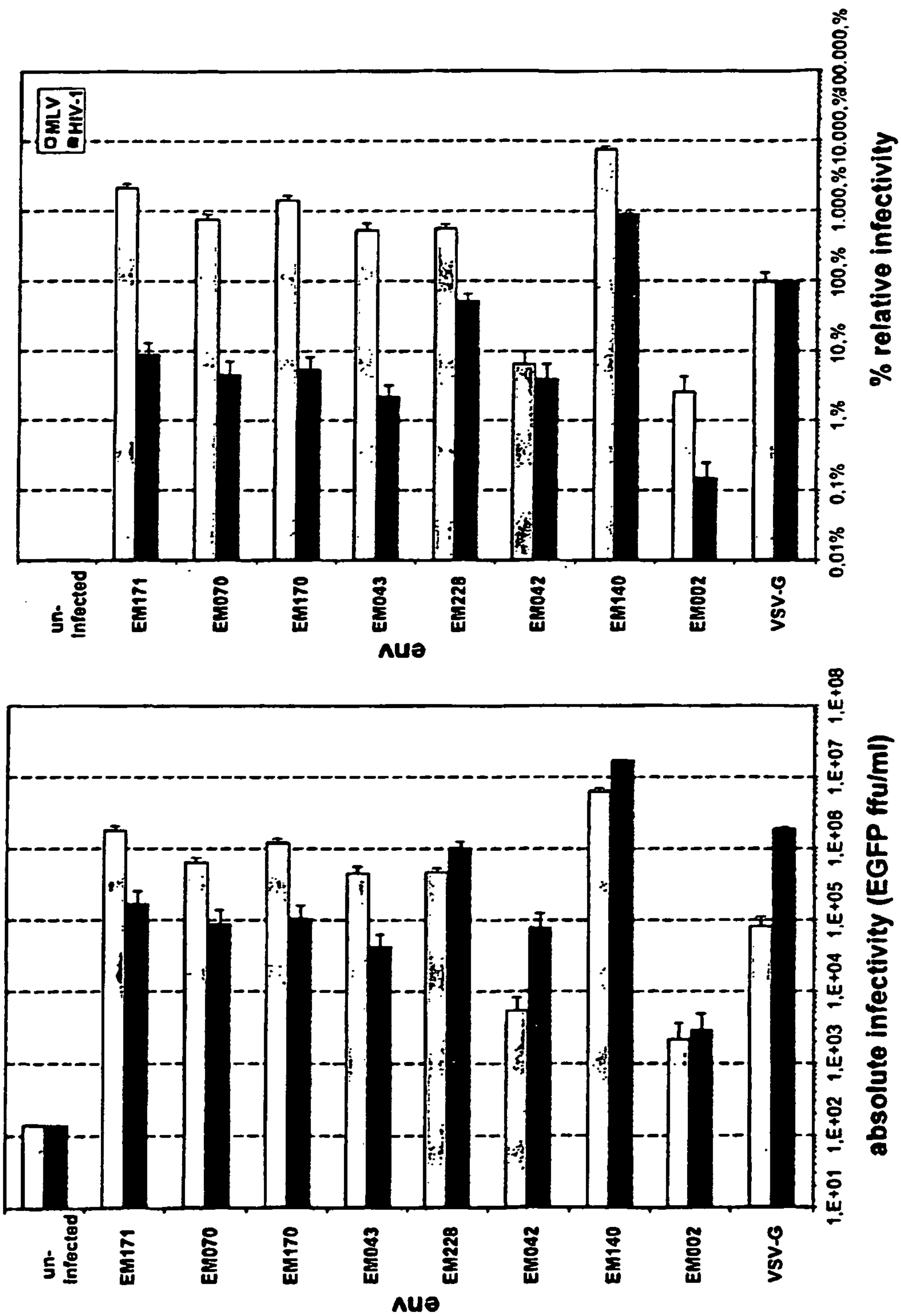
MLV
HIV-1
env
un-infected
EM171
EM070
EM170
EM043
EM228
EM042
EM140
EM002
VSV-G
0,01% 0,1% 1,% 10,% 100,% 1.000,% 10.000,% 100.000,%
% relative infectivity
1,E+01 1,E+02 1,E+03 1,E+04 1,E+05 1,E+06 1,E+07 1,E+08
absolute infectivity (EGFP ffu/ml)

Fig. 10

| Vector System | Envelope env | relative Infectivity | | absolute infectivity | |
|---|---|---|---|---|---|
| | | Mean | StdDev | Mean | StdDev |
| HIV-1 | VSV-G | 100,00% | 3,3% | 1,9E+06 | 6,4E+04 |
| | EM002 | 0,16% | 0,1% | 3,0E+03 | 1,9E+03 |
| | EM140 | 910,40% | | 1,8E+07 | |
| | EM042 | 4,14% | 2,3% | 8,0E+04 | 4,5E+04 |
| | EM228 | 53,53% | 11,7% | 1,0E+06 | 2,3E+05 |
| | EM043 | 2,24% | 1,0% | 4,3E+04 | 1,9E+04 |
| | EM170 | 5,59% | 2,6% | 1,1E+05 | 5,1E+04 |
| | EM070 | 4,67% | 2,5% | 9,0E+04 | 4,8E+04 |
| | EM171 | 9,05% | 4,1% | 1,7E+05 | 7,9E+04 |
| MLV | VSV-G | 100,00% | 32,8% | 8,3E+04 | 2,7E+04 |
| | EM002 | 2,64% | 1,7% | 2,2E+03 | 1,4E+03 |
| | EM140 | 7794,59% | 518,2% | 6,5E+06 | 4,3E+05 |
| | EM042 | 6,69% | 3,3% | 5,6E+03 | 2,7E+03 |
| | EM228 | 573,83% | 76,3% | 4,8E+05 | 6,4E+04 |
| | EM043 | 546,16% | 125,5% | 4,6E+05 | 1,0E+05 |
| | EM170 | 1468,81% | 185,9% | 1,2E+06 | 1,6E+05 |
| | EM070 | 786,23% | 110,6% | 6,6E+05 | 9,2E+04 |
| | EM171 | 2211,23% | 250,0% | 1,8E+06 | 2,1E+05 |
| | uninfected | 0,01% | 0,0% | 1,4E+02 | 0,0E+00 |

Fig. 11

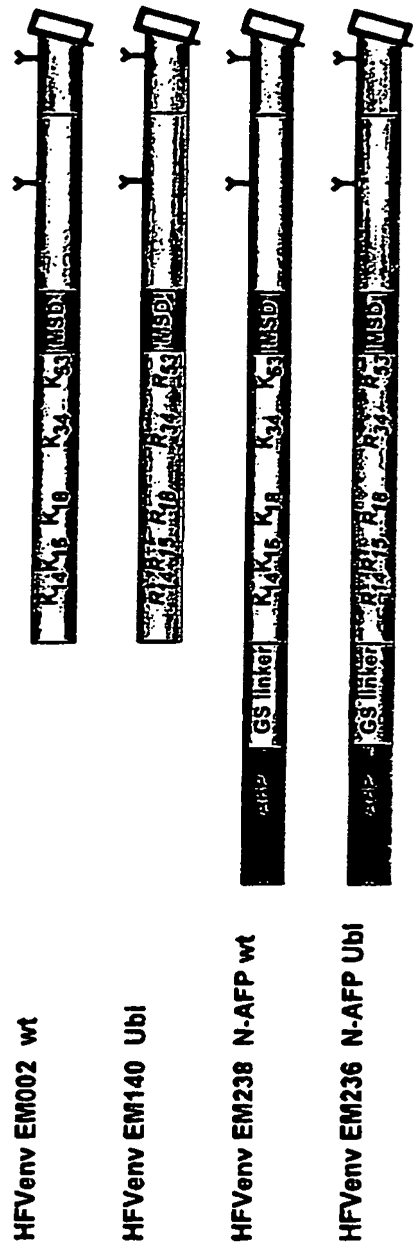
HFVenv EM002 wt
HFVenv EM140 Ubl
HFVenv EM238 N-AFP wt
HFVenv EM236 N-AFP Ubl
GS linker
MSD

Fig. 12

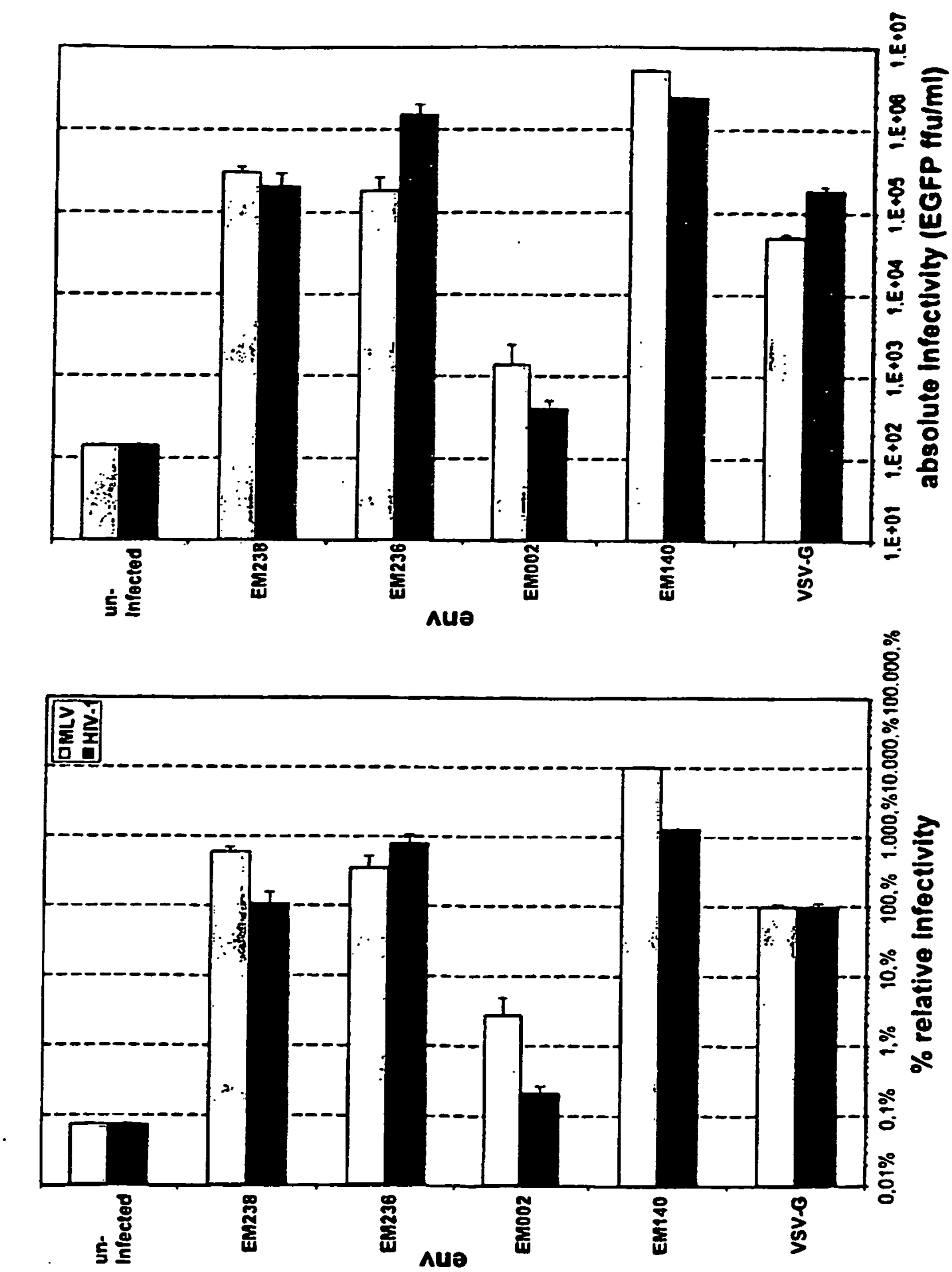
un-infected
EM238
EM236
EM002
EM140
VSV-G
env
1,E+01 1,E+02 1,E+03 1,E+04 1,E+05 1,E+06 1,E+07
absolute infectivity (EGFP ffu/ml)
MLV
HIV-1
0,01% 0,1% 1,% 10,% 100,% 1.000,% 10.000,% 100.000,%
% relative infectivity

Fig. 13

| Vector System | Envelope env | relative infectivity | | absolute infectivity | |
|---|---|---|---|---|---|
| | | Mean | StdDev | Mean | StdDev |
| HIV-1 | VSV-G | 100,00% | 9,3% | 1,9E+05 | 1,8E+04 |
| | EM140 | 1328,09% | | 2,5E+06 | |
| | EM002 | 0,22% | 0,1% | 4,1E+02 | 1,0E+02 |
| | EM236 | 813,61% | 262,4% | 1,5E+06 | 5,0E+05 |
| | EM238 | 110,39% | 46,4% | 2,1E+05 | 8,8E+04 |
| MLV | VSV-G | 100,00% | 6,3% | 5,1E+04 | 3,2E+03 |
| | EM140 | 10328,33% | | 5,3E+06 | |
| | EM002 | 2,80% | 2,0% | 1,4E+03 | 1,0E+03 |
| | EM236 | 359,02% | 161,0% | 1,8E+05 | 8,2E+04 |
| | EM238 | 607,39% | 88,1% | 3,1E+05 | 4,5E+04 |
| | uninfected | 0,08% | 0,0% | 1,4E+02 | 0,0E+00 |

Fig. 14

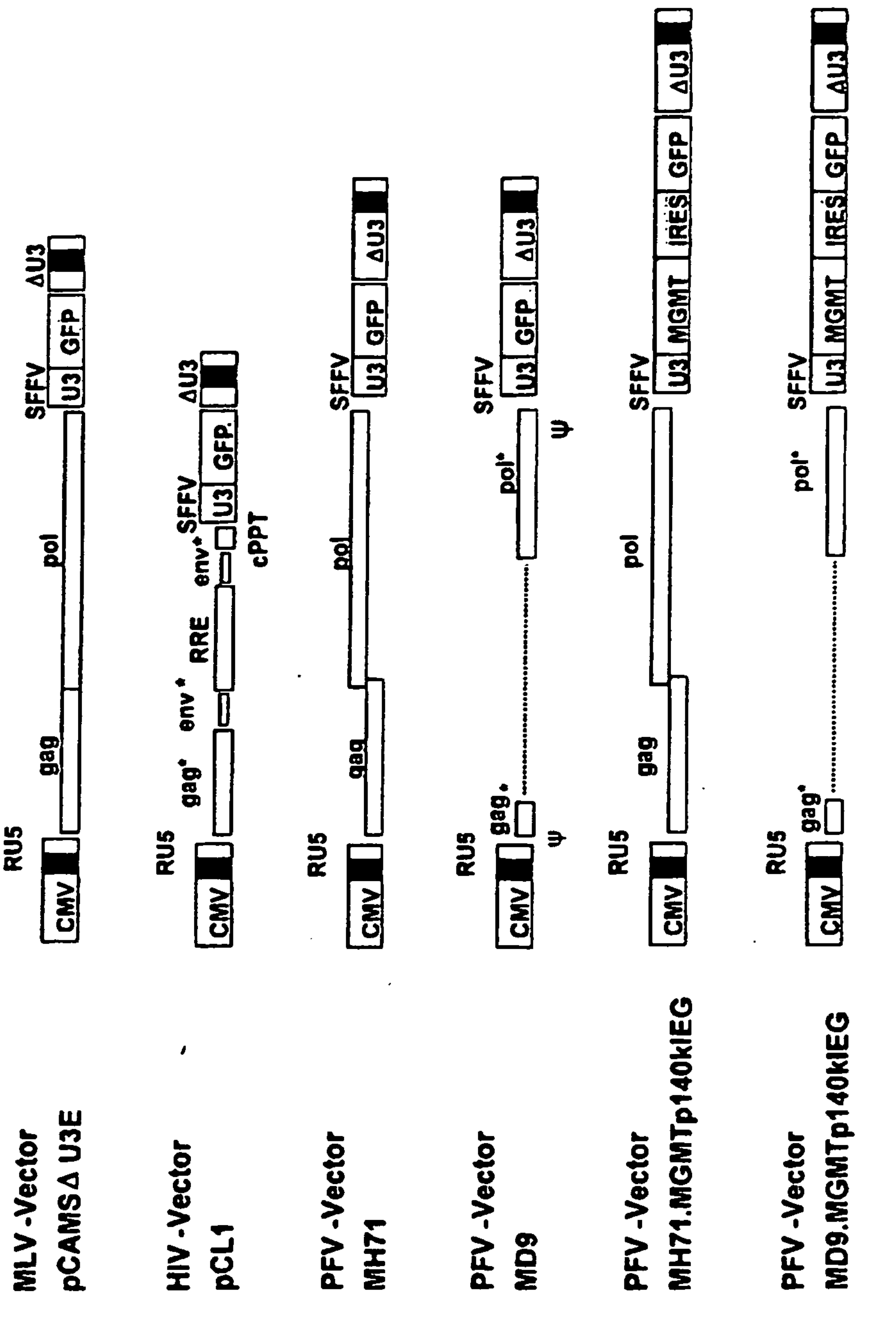
MLV-Vector
pCAMSΔ U3E
CMV
RU5
gag
pol
SFFV
U3
GFP
ΔU3
HIV-Vector
pCL1
gag*
env*
RRE
env*
cPPT
PFV-Vector
MH71
PFV-Vector
MD9
pol*
Ψ
PFV-Vector
MH71.MGMTp140kIEG
MGMT
IRES
PFV-Vector
MD9.MGMTp140kIEG

Fig. 15

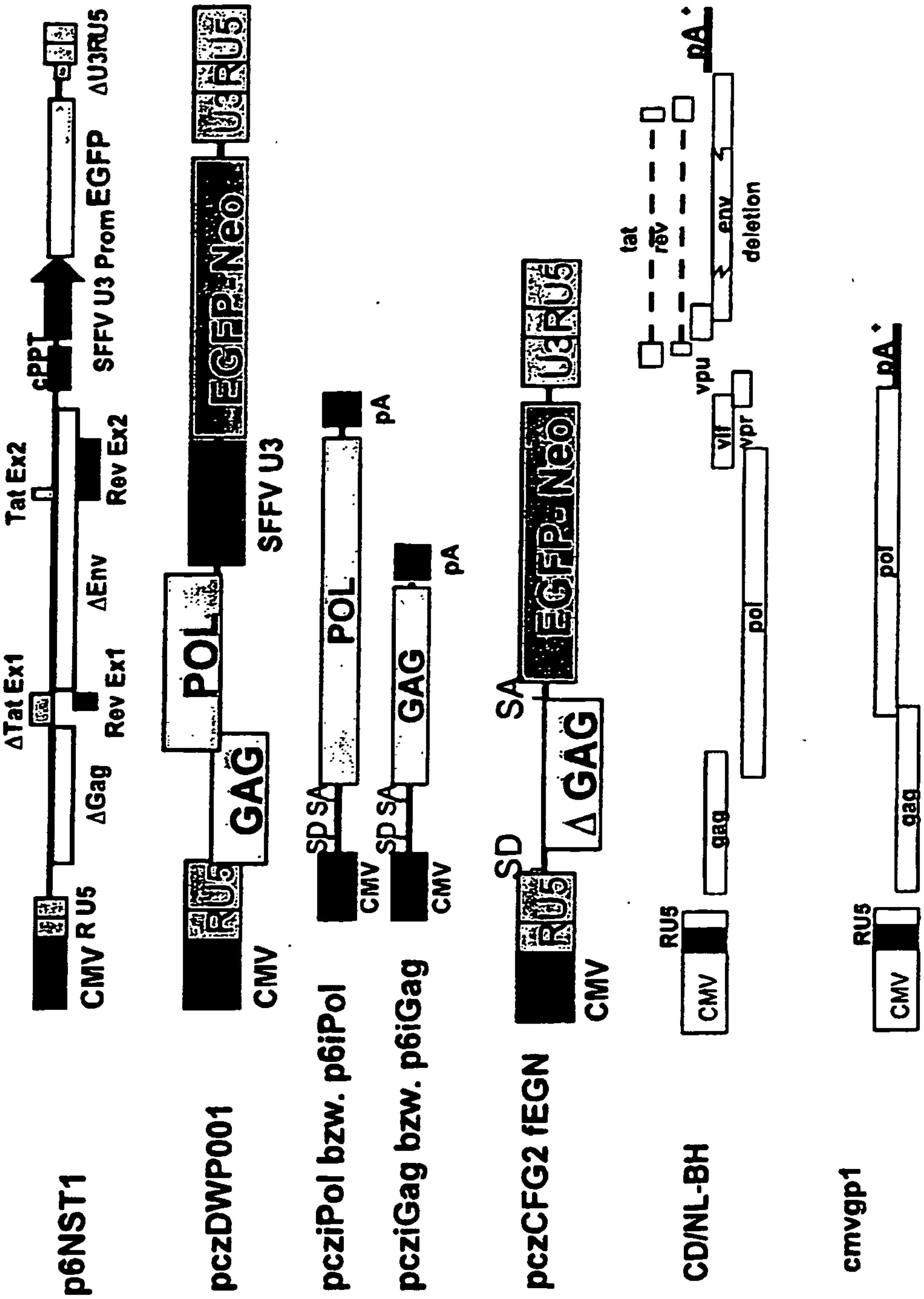
p6NST1
CMV R U5
ΔGag
ΔTat Ex1
Rev Ex1
ΔEnv
Tat Ex2
Rev Ex2
cPPT
SFFV U3 Prom
EGFP
ΔU3RU5
pczDWP001
CMV
RU5
GAG
POL
SFFV U3
EGFP-Neo
U3RU5
pcziPol bzw. p6iPol
CMV
SD SA
POL
pA
pcziGag bzw. p6iGag
CMV
SD SA
GAG
pA
pczCFG2 fEGN
CMV
RU5
SD
Δ GAG
SA
EGFP-Neo
U3RU5
CD/NL-BH
CMV
RU5
gag
pol
vif
vpr
vpu
tat
rev
env
deletion
pA
cmvgp1
CMV
RU5
gag
pol
pA

Fig. 16

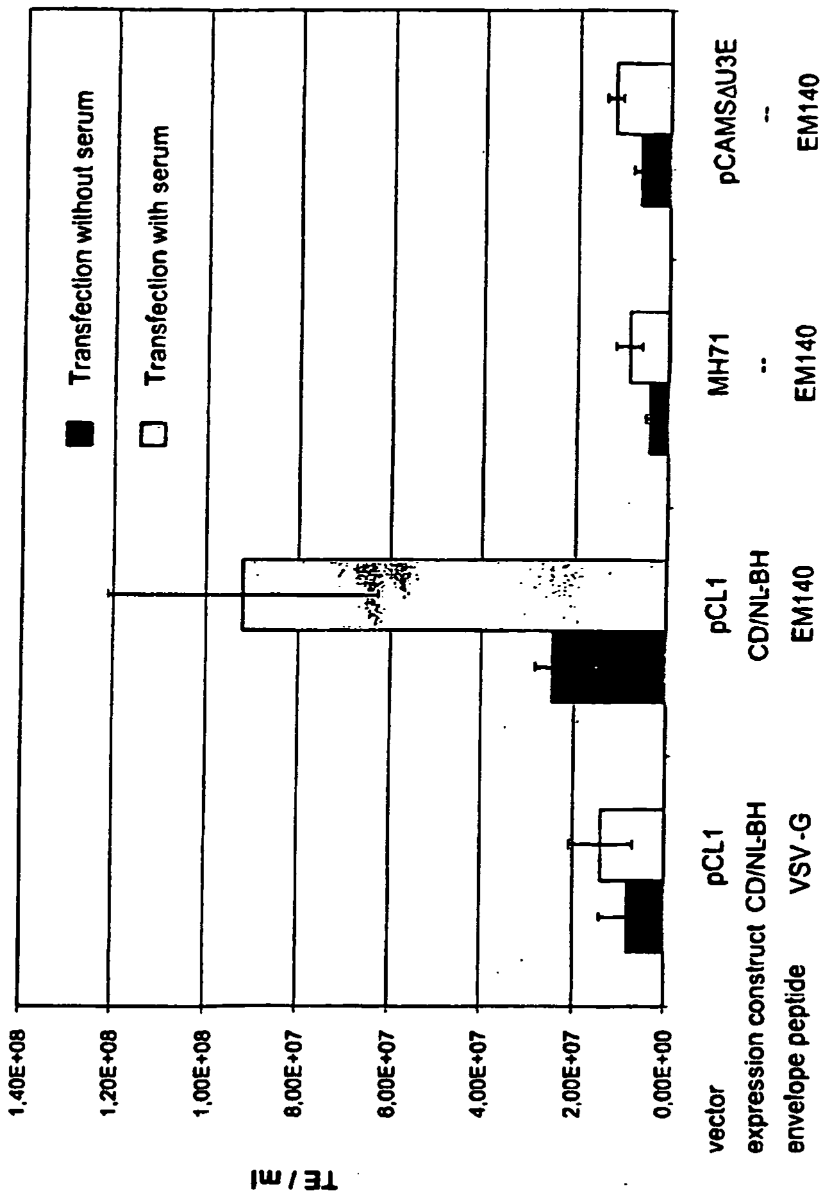
TE / ml
1,40E+08
1,20E+08
1,00E+08
8,00E+07
6,00E+07
4,00E+07
2,00E+07
0,00E+00
Transfection without serum
Transfection with serum
vector
expression construct
envelope peptide
pCL1
CD/NL-BH
VSV-G
pCL1
CD/NL-BH
EM140
MH71
--
EM140
pCAMSΔU3E
--
EM140

Fig. 17

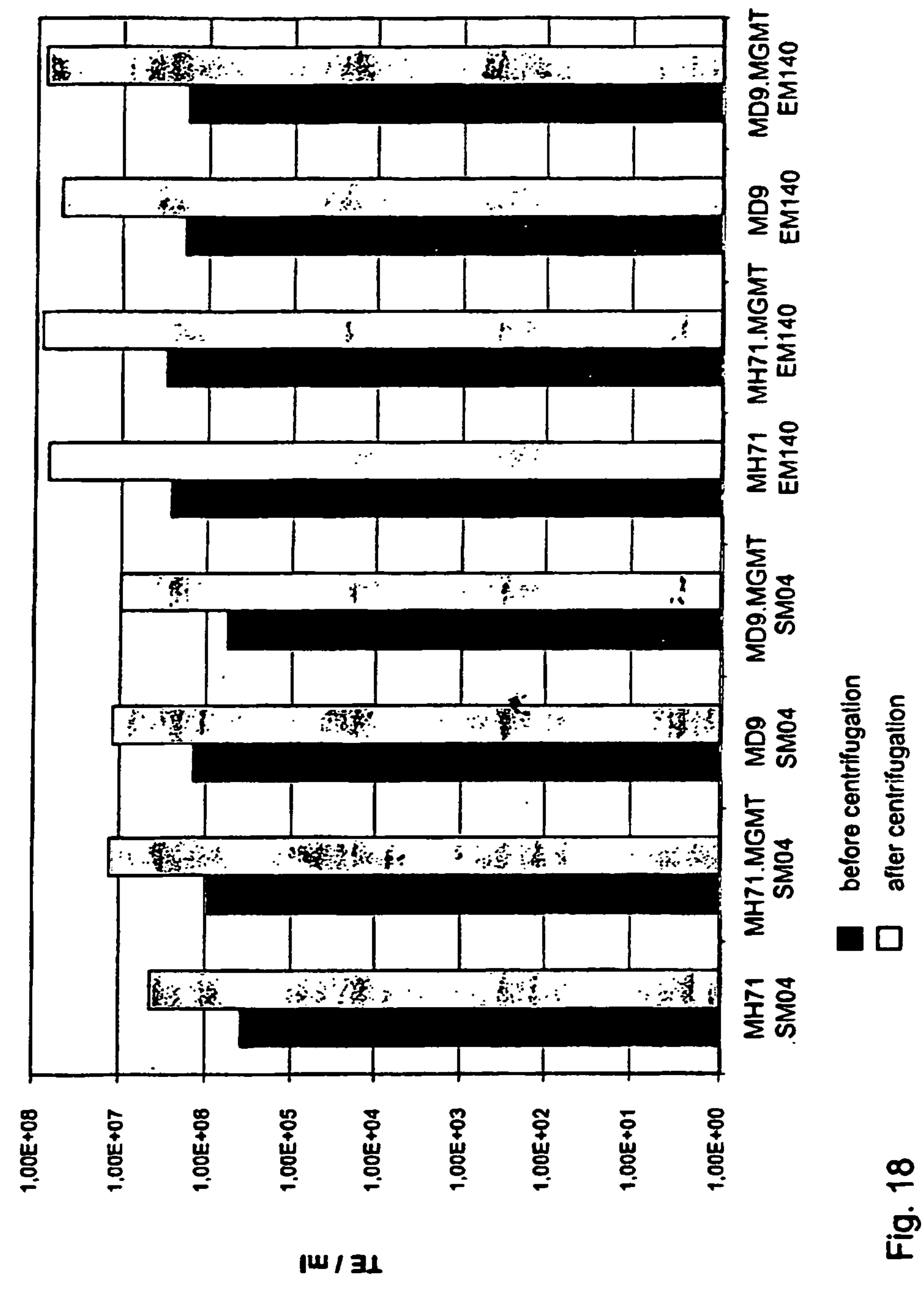
TE / ml
1,00E+08
1,00E+07
1,00E+06
1,00E+05
1,00E+04
1,00E+03
1,00E+02
1,00E+01
1,00E+00
MH71 SM04
MH71.MGMT SM04
MD9 SM04
MD9.MGMT SM04
MH71 EM140
MH71.MGMT EM140
MD9 EM140
MD9.MGMT EM140
before centrifugation
after centrifugation

Fig. 18

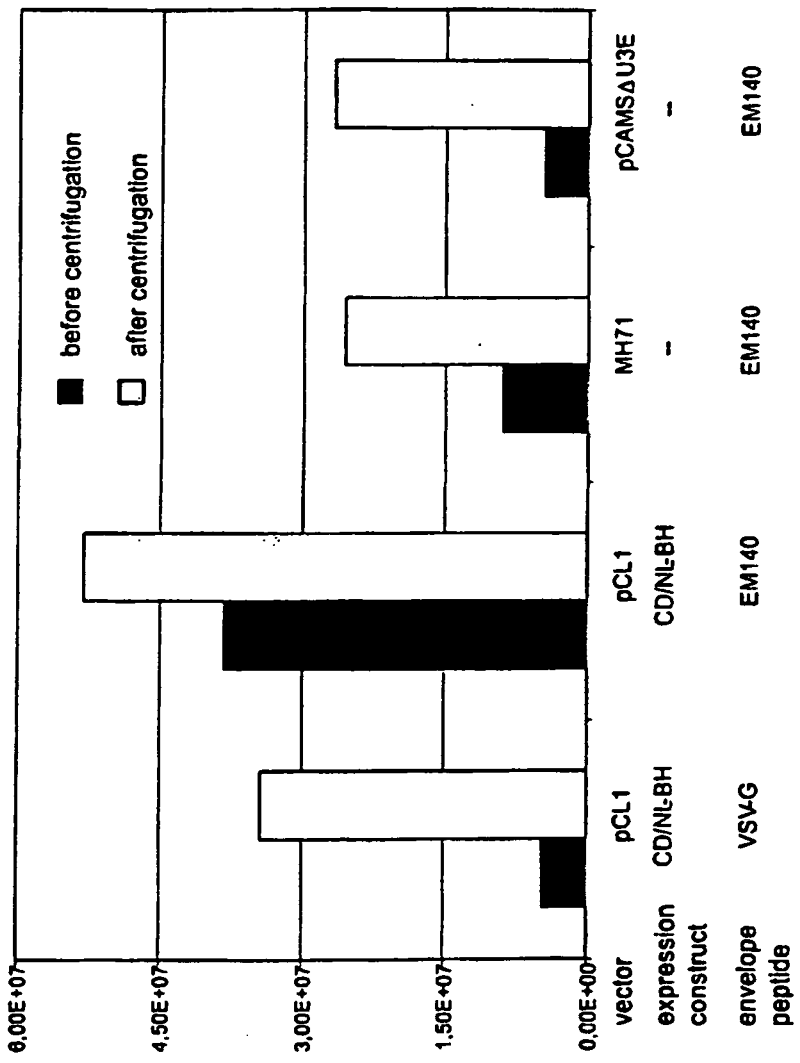
TE / ml
6,00E+07
4,50E+07
3,00E+07
1,50E+07
0,00E+00
before centrifugation
after centrifugation
vector
expression construct
envelope peptide
pCL1 CD/NL-BH
VSV-G
pCL1 CD/NL-BH
EM140
MH71
-
EM140
pCAMSΔU3E
-
EM140

Fig. 19

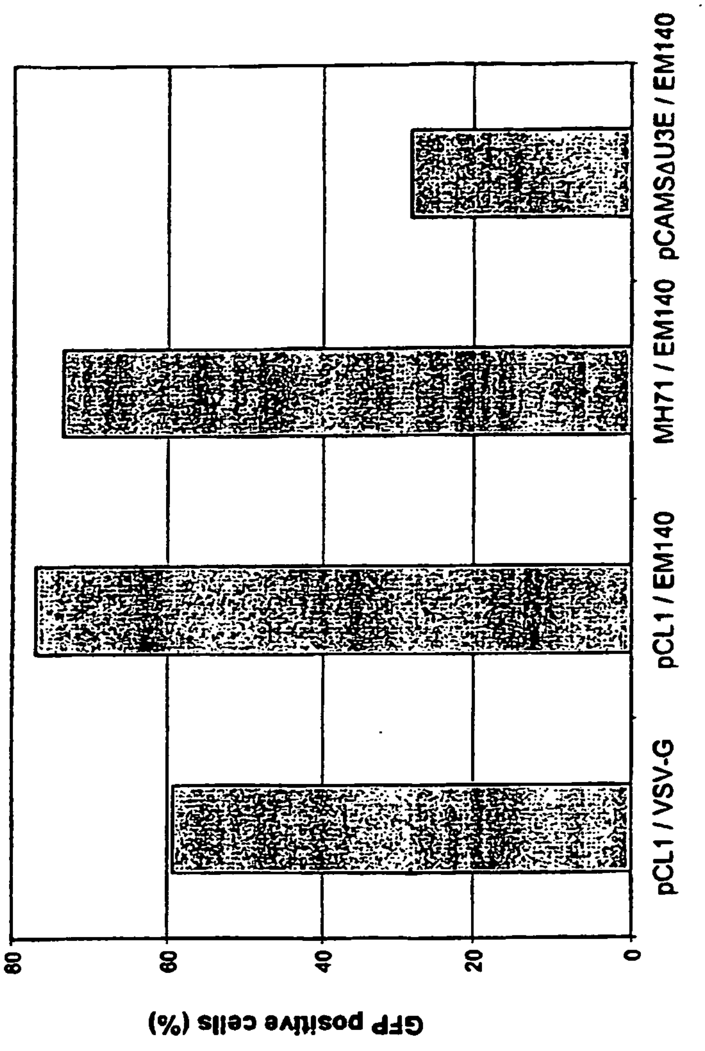
GFP positive cells (%)
0
20
40
60
80
pCL1 / VSV-G
pCL1 / EM140
MH71 / EM140
pCAMSΔU3E / EM140

Fig. 20

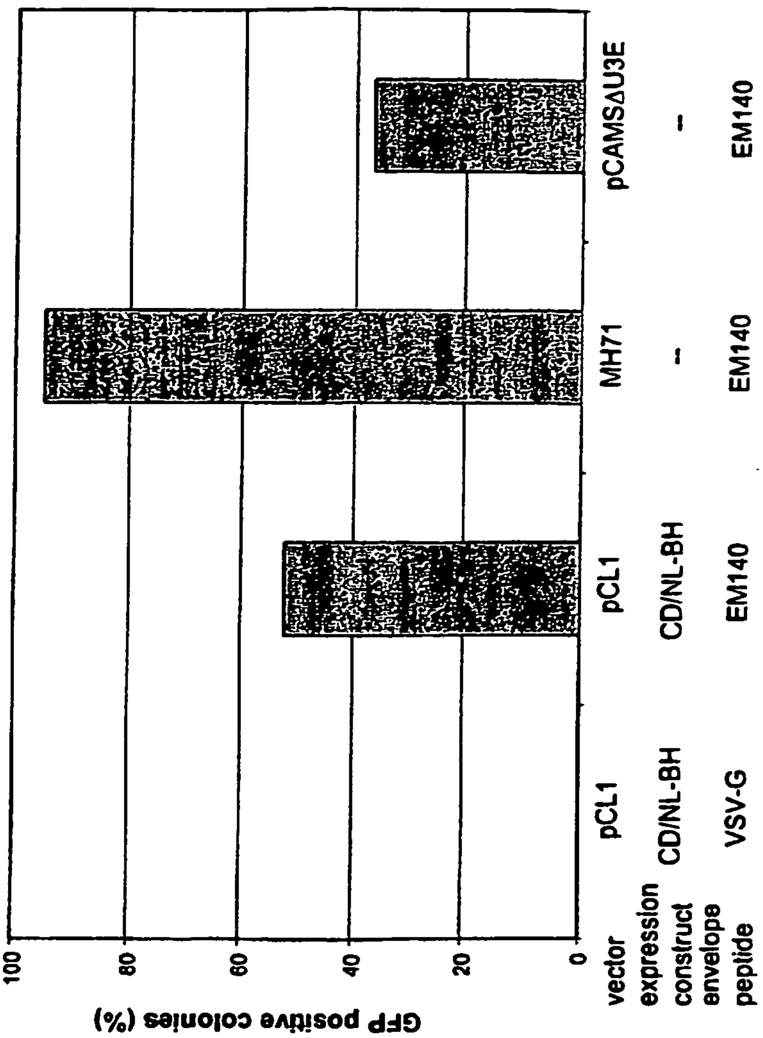
GFP positive colonies (%)
100
80
60
40
20
0
vector
expression construct
envelope peptide
pCL1
CD/NL-BH
VSV-G
pCL1
CD/NL-BH
EM140
MH71
--
EM140
pCAMSΔU3E
--
EM140

Fig. 21

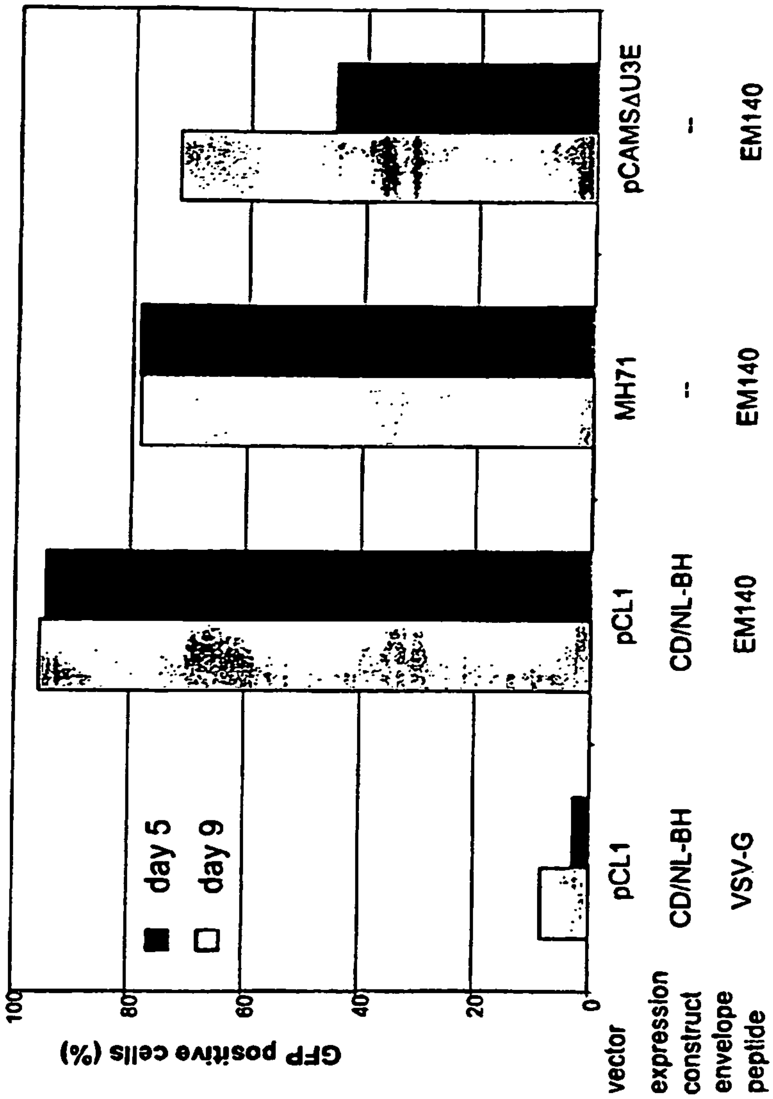
GFP positive cells (%)
100
80
60
40
20
0
day 5
day 9
vector
expression construct
envelope peptide
pCL1
CD/NL-BH
VSV-G
pCL1
CD/NL-BH
EM140
MH71
--
EM140
pCAMSΔU3E
--
EM140

Fig. 22

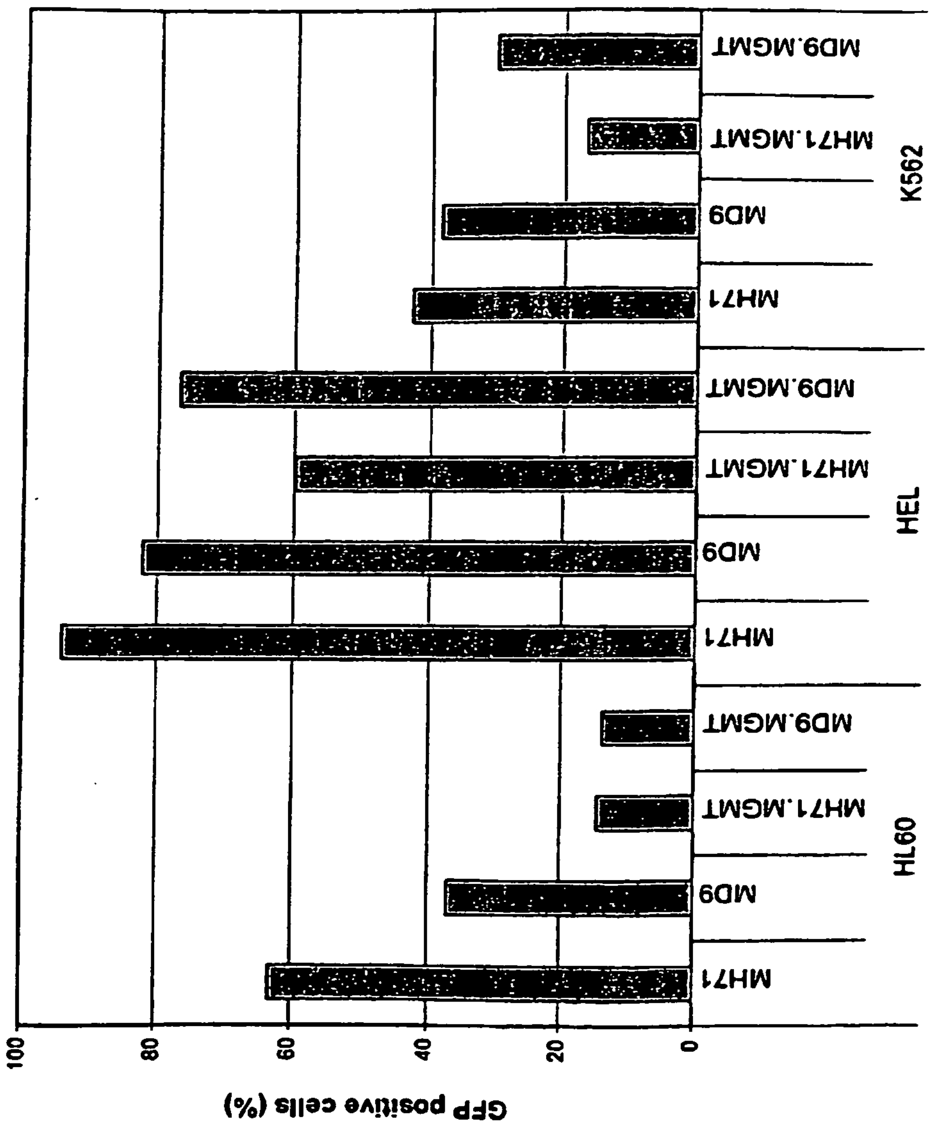
GFP positive cells (%)
100
80
60
40
20
0
MH71
MD9
MH71.MGMT
MD9.MGMT
HL60
HEL
K562

Fig. 23

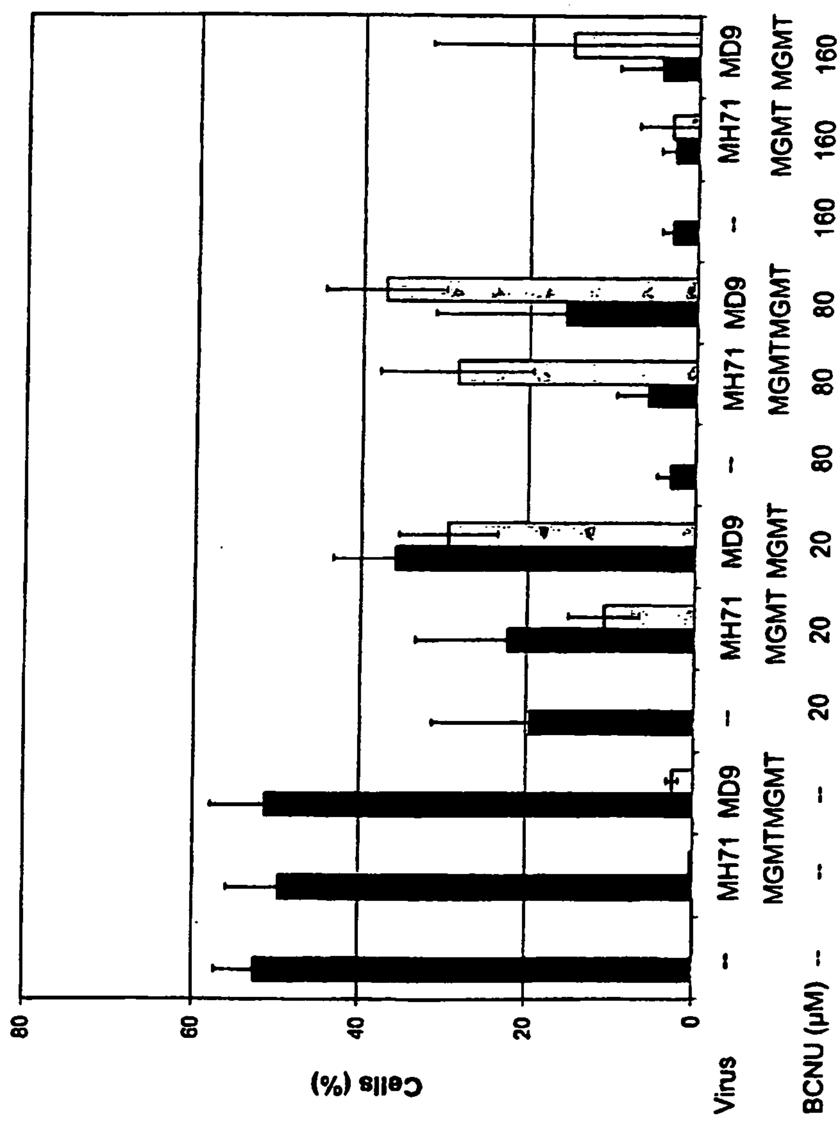
Cells (%)
0
20
40
60
80
Virus
--
MH71 MGMT
MD9 MGMT
--
MH71 MGMT
MD9 MGMT
--
MH71 MGMT
MD9 MGMT
--
MH71 MGMT
MD9 MGMT
BCNU (µM)
--
--
--
20
20
20
80
80
80
160
160
160

Fig. 24

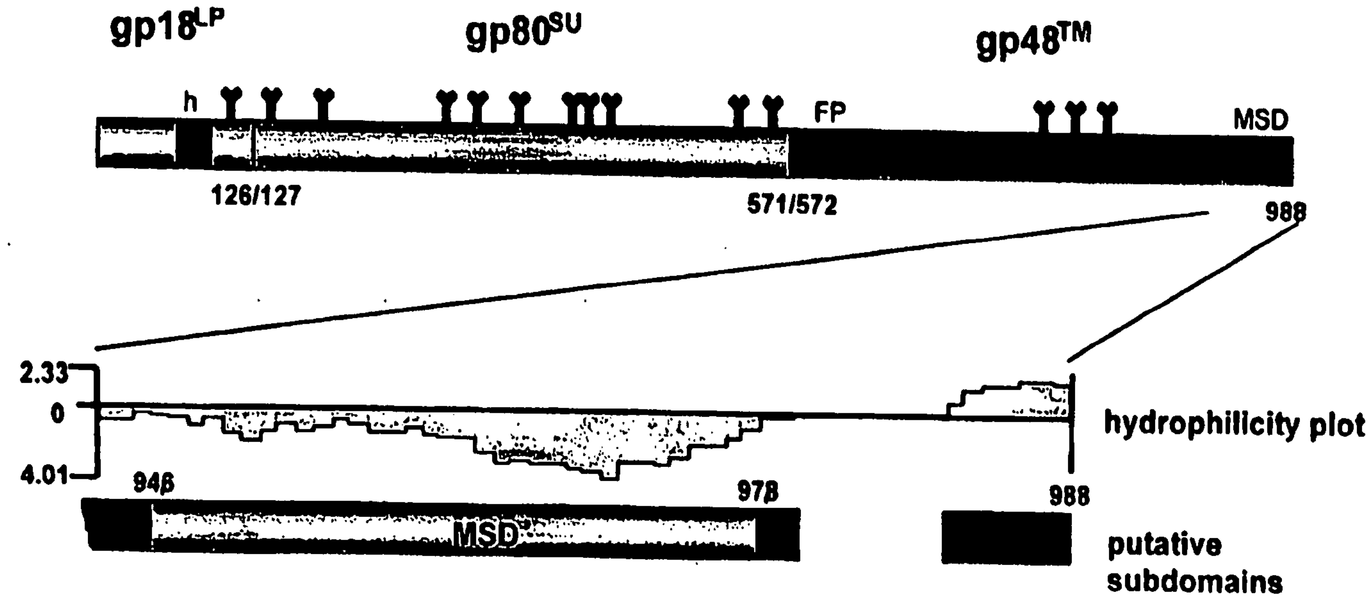


| | | | |
|---|---|---|---|
| 946 | AQGIFGTAFSLLGYLKPILIGVGVILLVILIFKIVSWI--------PTKKKNQ | 988 | PFV |
| 946 | AHGIFGTAFSLLGYLKPILIGVGVILLIILIFKIVSWI--------PTKKKSQ | 988 | SFVcpz |
| 947 | AQGIFGSAVSLLFYAKPILIGIGVILLIALLFKIISWL--------PGKPKKN | 989 | SFVmac |
| 940 | AQGIFGTTVSILSYAKPILIGIGVILLIAFLFKIVSWL--------PGKKKRN | 982 | SFVagm |
| 947 | AQGIFGTAFGILGYVKPILIGVGIILLIVVIFKIISWI--------PIKRKSQ | 989 | SFVora |
| 944 | LSGTFGGLFGTLGYIKPIIVGIVILLLIVIVCKILSWL--------PSKRKTQ | 986 | SFVspm |
| 946 | AGTLFGNVFSILAYAKPVIIGIILIILLLLVIRILRWLAV-------ERRRKQE | 990 | BFV |
| 936 | ADGIFGTTFSLLTYAKPVIIGIVVIVLLILIIRILSWLAALGPRRRRREDKGE | 986 | EFV |
| 937 | AGGIFGTAFSFLGYVKPVLLGFVIIFCIILIIKIIGWL-------QNTRKKDQ | 980 | FFV |

Fig. 25

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5929222 A
- US 6111087 A


### Non-patent literature cited in the description


- **ANDERSON et al.** *Hum. Gene. Ther.,* 1990, vol. 1 (3), 331-62
- **CHECK.** *Nature,* 2005, vol. 433, 561
- **NIENHUIS et al.** *Ann. N. Y. Acad. Sci.,* 2003, vol. 996, 101-11
- **LI et al.** *J. Hum. Virol.,* 1998, vol. 1 (5), 346-52
- **LI et al.** *Science,* 2002, vol. 296, 497
- **LINDEMANN et al.** *J. Virol.,* 2001, vol. 75, 5762-5771
- **RUSSEL ; MILLER.** *J. Virol.,* 1996, vol. 70, 217-222
- **STANKE et al.** *J. Virol.,* 2005, vol. 79, 15074-15083
- **SCHMIDT ; RETHWILM.** *Virology,* 1995, vol. 210, 167-178
- **WEISS.** *Nature,* 1996, vol. 380, 201
- **WILK et al.** *J. Virol.,* 2001, vol. 74, 2885-2887

SEQUENCE LISTING

<110> Vectoria Forschungsförderungs Verein e.V.

<120> Foamy viral envelope gene

<130> V30006

<160> 39

<170> PatentIn version 3.3

<210> 7
<211> 2967
<212> DNA
<213> Foamyvirus

<400> 7

```
atggcccctc ccatgaccct gcagcagtgg atcatctggc ggcggatgaa ccgggcccac      60
gaggccctgc agaacaccac caccgtgacc gagcagcagc gggagcagat catcctggac     120
atccagaacg aggaagtgca gcccaccagg cgggaccggt tcagatacct gctgtacacc     180
tgctgcgcca cctccagccg ggtgctggcc tggatgttcc tggtgtgcat cctgctgatc     240
atcgtgctgg tgtcctgctt cgtgaccatc agccggatcc agtggaacaa ggacatccag     300
gtgctgggcc ccgtgatcga ctggaacgtg acccagcggg ccgtgtacca gcccctgcag     360
acccggcgga tcgcccggtc cctgcggatg cagcaccccg tgcccaagta cgtggaggtg     420
aacatgacca gcatccccca gggcgtgtac tacgagcccc accccgagcc catcgtggtg     480
aaagaaagag tgctgggcct gagccagatc ctgatgatca acagcgagaa catcgccaac     540
aacgccaacc tgacccagga agtgaagaaa ctgctgaccg agatggtgaa cgaagagatg     600
cagagcctga gcgacgtgat gatcgacttc gagatccccc tgggcgaccc cagggaccag     660
gaacagtaca tccaccggaa gtgctaccag gaatttgcca actgctacct ggtgaagtac     720
aaagagccca agccctggcc caaagagggc ctgatcgccg accagtgccc cctgcccggc     780
tatcacgccg gcctgaccta caaccggcag agcatctggg actactacat caaggtggag     840
agcatcaggc ccgccaactg gaccaccaag agcaagtacg gccaggcccg gctgggcagc     900
ttctacatcc ccagcagcct gcggcagatc aacgtgagcc acgtgctgtt ctgcagcgac     960
cagctgtaca gcaagtggta caacatcgag aacaccatcg agcagaacga gcggttcctg    1020
```

ctgaacaagc tgaataacct gaccagcggc accagcgtgc tgaagaagag agccctgccc 1080
aaggactggt ccagccaggg caagaacgcc ctgttccggg agatcaatgt gctggacatc 1140
tgcagcaagc ccgagagcgt gatcctgctg aataccagct actacagctt cagcctgtgg 1200
gagggcgact gcaacttcac caaggacatg atcagccagc tggtgcccga gtgcgacggc 1260
ttctacaaca actccaagtg gatgcacatg caccccctacg cctgccggtt ctggcggagc 1320
aagaacgaga aagaggaaac caagtgccgg gacggcgaga ccaagcggtg cctgtactac 1380
cccctgtggg acagccctga gagcacctac gacttcggct acctggccta ccagaagaac 1440
ttcccccagcc ccatctgcat cgaacagcag aagatccggg accaggacta cgaggtgtac 1500
agcctgtacc aggaatgcaa gatcgccagc aaggcctacg gcatcgacac cgtgctgttc 1560
agcctgaaga atttcctgaa ctacaccggc acccccgtga acgagatgcc caacgccagg 1620
gccttcgtgg gcctgattga ccccaagttc ccccccagct accccaacgt gacccgggag 1680
cactacacca gctgcaacaa ccggaagcgg cggagcgtgg acaacaacta cgccaagctg 1740
cggagcatgg gctacgctct gacaggcgcc gtgcagaccc tgtcccagat cagcgacatc 1800
aacgacgaga acctgcagca gggcatctac ctgctgcggg accacgtgat caccctgatg 1860
gaagccaccc tgcacgacat cagcgtgatg gaaggcatgt tcgccgtgca gcacctgcac 1920
acccacctga atcacctgaa aaccatgctg ctggaacggc gcatcgactg gacctacatg 1980
agcagcacct ggctgcagca gcagctgcag aaaagcgacg acgagatgaa ggtgatcaag 2040
cggatcgcca gatctctggt gtactacgtg aagcagaccc acagcagccc caccgccacc 2100
gcctgggaga tcggcctgta ctatgagctg gtgatcccca agcacatcta cctgaacaac 2160
tggaatgtgg tgaacatcgg ccacctggtg aaaagcgccg gacagctgac ccacgtgacc 2220
atcgcccacc cctacgagat catcaacaaa gaatgcgtgg agaccatcta tctgcacctg 2280
gaagattgca cccggcagga ctacgtgatc tgcgacgtgg tgaagatcgt gcagccctgc 2340
ggcaacagca gcgacaccag cgactgcccc gtgtgggccg aggccgtgaa agaacccttc 2400
gtgcaggtga acccccctgaa gaacggctcc tacctggtgc tggccagcag caccgactgc 2460
cagatccccc cctacgtgcc cagcatcgtg accgtgaatg agaccacctc ctgcttcggc 2520
ctggacttca agcggcccct ggtggccgag gaaagactga gcttcgagcc ccggctgccc 2580
aacctgcagc tgaggctgcc ccacctggtg ggcatcatcg ccaagatcaa gggcatcaag 2640
atcgaggtga ccagcagcgg cgagagcatc aaagaacaga tcgagcgggc caaggccgag 2700
ctgctgcggc tggatatcca cgagggcgac acacccgcct ggatccagca gctggccgcc 2760
gccaccaagg acgtgtggcc cgctgcagcc agcgccctgc agggcatcgg caactttctg 2820
agcggcaccg cccagggcat cttcggcacc gccttctccc tgctgggcta cctgaagccc 2880

atcctgatcg gcgtgggcgt gattctgctg gtgattctga tcttcaagat cgtgagctgg 2940

atccccacca agaaaaagaa ccagtga 2967

<210> 8
<211> 2967
<212> DNA
<213> Foamyvirus

<400> 8

atggcccctc ccatgaccct gcagcagtgg atcatctggc ggcggatgaa ccgggcccac 60

gaggccctgc agaacaccac caccgtgacc gagcagcagc gggagcagat catcctggac 120

atccagaacg aggaagtgca gcccaccagg cgggaccggt tcagatacct gctgtacacc 180

tgctgcgcca cctccagccg ggtgctggcc tggatgttcc tggtgtgcat cctgctgatc 240

atcgtgctgg tgtcctgctt cgtgaccatc agccggatcc agtggaacaa ggacatccag 300

gtgctgggcc ccgtgatcga ctggaacgtg acccagcggg ccgtgtacca gcccctgcag 360

acccggcgga tcgcccggtc cctgcggatg cagcaccccg tgcccaagta cgtggaggtg 420

aacatgacca gcatccccca gggcgtgtac tacgagcccc accccgagcc catcgtggtg 480

aaagaaagag tgctgggcct gagccagatc ctgatgatca acagcgagaa catcgccaac 540

aacgccaacc tgacccagga agtgaagaaa ctgctgaccg agatggtgaa cgaagagatg 600

cagagcctga gcgacgtgat gatcgacttc gagatccccc tgggcgaccc cagggaccag 660

gaacagtaca tccaccggaa gtgctaccag gaatttgcca actgctacct ggtgaagtac 720

aaagagccca agccctggcc caaagagggc ctgatcgccg accagtgccc cctgcccggc 780

tatcacgccg gcctgaccta caaccggcag agcatctggg actactacat caaggtggag 840

agcatcaggc ccgccaactg gaccaccaag agcaagtacg gccaggcccg gctgggcagc 900

ttctacatcc ccagcagcct gcggcagatc aacgtgagcc acgtgctgtt ctgcagcgac 960

cagctgtaca gcaagtggta caacatcgag aacaccatcg agcagaacga gcggttcctg 1020

ctgaacaagc tgaataacct gaccagcggc accagcgtgc tgaagaagag agccctgccc 1080

aaggactggt ccagccaggg caagaacgcc ctgttccggg agatcaatgt gctggacatc 1140

tgcagcaagc ccgagagcgt gatcctgctg aataccagct actacagctt cagcctgtgg 1200

gagggcgact gcaacttcac caaggacatg atcagccagc tggtgcccga gtgcgacggc 1260

ttctacaaca actccaagtg gatgcacatg cacccctacg cctgccggtt ctggcggagc 1320

aagaacgaga aagaggaaac caagtgccgg gacggcgaga ccaagcggtg cctgtactac 1380

cccctgtggg acagccctga gagcacctac gacttcggct acctggccta ccagaagaac 1440

ttccccagcc ccatctgcat cgaacagcag aagatccggg accaggacta cgaggtgtac 1500

agcctgtacc aggaatgcaa gatcgccagc aaggcctacg gcatcgacac cgtgctgttc 1560

agcctgaaga atttcctgaa ctacaccggc acccccgtga acgagatgcc caacgccagg 1620
gccttcgtgg gcctgattga ccccaagttc ccccccagct accccaacgt gacccgggag 1680
cactacacca gctgcaacaa ccggaagcgg cggagcgtgg acaacaacta cgccaagctg 1740
cggagcatgg gctacgctct gacaggcgcc gtgcagaccc tgtcccagat cagcgacatc 1800
aacgacgaga acctgcagca gggcatctac ctgctgcggg accacgtgat caccctgatg 1860
gaagccaccc tgcacgacat cagcgtgatg gaaggcatgt tcgccgtgca gcacctgcac 1920
acccacctga atcacctgaa aaccatgctg ctggaacggc gcatcgactg gacctacatg 1980
agcagcacct ggctgcagca gcagctgcag aaaagcgacg acgagatgaa ggtgatcaag 2040
cggatcgcca gatctctggt gtactacgtg aagcagaccc acagcagccc caccgccacc 2100
gcctgggaga tcggcctgta ctatgagctg gtgatcccca agcacatcta cctgaacaac 2160
tggaatgtgg tgaacatcgg ccacctggtg aaaagcgccg gacagctgac ccacgtgacc 2220
atcgcccacc cctacgagat catcaacaaa gaatgcgtgg agaccatcta tctgcacctg 2280
gaagattgca cccggcagga ctacgtgatc tgcgacgtgg tgaagatcgt gcagccctgc 2340
ggcaacagca gcgacaccag cgactgcccc gtgtgggccg aggccgtgaa agaacccttc 2400
gtgcaggtga acccccctgaa gaacggctcc tacctggtgc tggccagcag caccgactgc 2460
cagatccccc cctacgtgcc cagcatcgtg accgtgaatg agaccacctc ctgcttcggc 2520
ctggacttca agcggcccct ggtggccgag gaaagactga gcttcgagcc ccggctgccc 2580
aacctgcagc tgaggctgcc ccacctggtg ggcatcatcg ccaagatcaa gggcatcaag 2640
atcgaggtga ccagcagcgg cgagagcatc aaagaacaga tcgagcgggc caaggccgag 2700
ctgctgcggc tggatatcca cgagggcgac acacccgcct ggatccagca gctggccgcc 2760
gccaccaagg acgtgtggcc cgctgcagcc agcgccctgc agggcatcgg caactttctg 2820
agcggcaccg cccagggcat cttcggcacc gccttctccc tgctgggcta cctgaagccc 2880
atcctgatcg gcgtgggcgt gattctgctg gtgattctga tcttcaagat cgtgagctgg 2940
atccccaccc ggagacggaa ccagtga 2967

<210> 9
<211> 2967
<212> DNA
<213> Foamyvirus

<400> 9

atggcccctc ccatgaccct gcagcagtgg atcatctggc ggcggatgaa ccgggcccac 60
gaggccctgc agaacaccac caccgtgacc gagcagcagc gggagcagat catcctggac 120
atccagaacg aggaagtgca gcccaccagg cgggaccggt tcagatacct gctgtacacc 180
tgctgcgcca cctccagccg ggtgctggcc tggatgttcc tggtgtgcat cctgctgatc 240

atcgtgctgg tgtcctgctt cgtgaccatc agccggatcc agtggaacaa ggacatccag 300
gtgctgggcc ccgtgatcga ctggaacgtg acccagcggg ccgtgtacca gcccctgcag 360
acccggcgga tcgcccggtc cctgcggatg cagcaccccg tgcccaagta cgtggaggtg 420
aacatgacca gcatccccca gggcgtgtac tacgagcccc accccgagcc catcgtggtg 480
aaagaaagag tgctgggcct gagccagatc ctgatgatca acagcgagaa catcgccaac 540
aacgccaacc tgacccagga agtgaagaaa ctgctgaccg agatggtgaa cgaagagatg 600
cagagcctga gcgacgtgat gatcgacttc gagatccccc tgggcgaccc cagggaccag 660
gaacagtaca tccaccggaa gtgctaccag gaatttgcca actgctacct ggtgaagtac 720
aaagagccca agccctggcc caaagagggc ctgatcgccg accagtgccc cctgcccggc 780
tatcacgccg gcctgaccta caaccggcag agcatctggg actactacat caaggtggag 840
agcatcaggc ccgccaactg gaccaccaag agcaagtacg gccaggcccg gctgggcagc 900
ttctacatcc ccagcagcct gcggcagatc aacgtgagcc acgtgctgtt ctgcagcgac 960
cagctgtaca gcaagtggta caacatcgag aacaccatcg agcagaacga gcggttcctg 1020
ctgaacaagc tgaataacct gaccagcggc accagcgtgc tgaagaagag agccctgccc 1080
aaggactggt ccagccaggg caagaacgcc ctgttccggg agatcaatgt gctggacatc 1140
tgcagcaagc ccgagagcgt gatcctgctg aataccagct actacagctt cagcctgtgg 1200
gagggcgact gcaacttcac caaggacatg atcagccagc tggtgcccga gtgcgacggc 1260
ttctacaaca actccaagtg gatgcacatg cacccctacg cctgccggtt ctggcggagc 1320
aagaacgaga aagaggaaac caagtgccgg gacggcgaga ccaagcggtg cctgtactac 1380
cccctgtggg acagccctga gagcacctac gacttcggct acctggccta ccagaagaac 1440
ttccccagcc ccatctgcat cgaacagcag aagatccggg accaggacta cgaggtgtac 1500
agcctgtacc aggaatgcaa gatcgccagc aaggcctacg gcatcgacac cgtgctgttc 1560
agcctgaaga atttcctgaa ctacaccggc acccccgtga acgagatgcc caacgccagg 1620
gccttcgtgg gcctgattga ccccaagttc ccccccagct accccaacgt gacccgggag 1680
cactacacca gctgcaacaa ccggaagcgg cggagcgtgg acaacaacta cgccaagctg 1740
cggagcatgg gctacgctct gacaggcgcc gtgcagaccc tgtcccagat cagcgacatc 1800
aacgacgaga acctgcagca gggcatctac ctgctgcggg accacgtgat caccctgatg 1860
gaagccaccc tgcacgacat cagcgtgatg gaaggcatgt tcgccgtgca gcacctgcac 1920
acccacctga atcacctgaa aaccatgctg ctggaacggc gcatcgactg gacctacatg 1980
agcagcacct ggctgcagca gcagctgcag aaaagcgacg acgagatgaa ggtgatcaag 2040
cggatcgcca gatctctggt gtactacgtg aagcagaccc acagcagccc caccgccacc 2100
gcctgggaga tcggcctgta ctatgagctg gtgatcccca agcacatcta cctgaacaac 2160

tggaatgtgg tgaacatcgg ccacctggtg aaaagcgccg gacagctgac ccacgtgacc 2220
atcgcccacc cctacgagat catcaacaaa gaatgcgtgg agaccatcta tctgcacctg 2280
gaagattgca cccggcagga ctacgtgatc tgcgacgtgg tgaagatcgt gcagccctgc 2340
ggcaacagca gcgacaccag cgactgcccc gtgtgggccg aggccgtgaa agaacccttc 2400
gtgcaggtga accccctgaa gaacggctcc tacctggtgc tggccagcag caccgactgc 2460
cagatccccc cctacgtgcc cagcatcgtg accgtgaatg agaccacctc ctgcttcggc 2520
ctggacttca agcggcccct ggtggccgag gaaagactga gcttcgagcc ccggctgccc 2580
aacctgcagc tgaggctgcc ccacctggtg ggcatcatcg ccaagatcaa gggcatcaag 2640
atcgaggtga ccagcagcgg cgagagcatc aaagaacaga tcgagcgggc caaggccgag 2700
ctgctgcggc tggatatcca cgagggcgac acacccgcct ggatccagca gctggccgcc 2760
gccaccaagg acgtgtggcc cgctgcagcc agcgccctgc agggcatcgg caactttctg 2820
agcggcaccg cccagggcat cttcggcacc gccttctccc tgctgggcta cctgcggccc 2880
atcctgatcg gcgtgggcgt gattctgctg gtgattctga tcttccggat cgtgagctgg 2940
atccccaccc ggagacggaa ccagtga 2967

<210> 10
<211> 2970
<212> DNA
<213> Foamyvirus

<400> 10

atggcccctc ccatgaccct ggaacagtgg ctgctgtggc ggcggatgag ccaggcccac 60
caggccctgg aaaacgtgac caccctgacc gaggaacagc ggcagcaggt gatcatcgac 120
atccagcacg aggacgtggt gcccacccgg atggaccggc tgcggtacct ggcctacagc 180
tgctgcgcca cctccacccg ggtgctgtgc tggatcgtgc tggtgtgcgt gctgctgctg 240
gtggtgttca tcagctgctt cgtgaccatg agccggatcc agtggaacaa ggacatcgcc 300
gtgttcggcc ccgtgatcga ctggaacgtg agccagcagg ccgtgatcca gcagatccgg 360
gccaagcggc tggccagatc catccgggtc gagcacgcca ccgagaccta cgtggaggtg 420
aacatgacca gcatccccca gggcgtgctg tacgtgcccc accccgagcc catcatcctg 480
aaagaacggg tgctgggcct gagccaggtg atcatgatca acagcgagaa catcgccaac 540
accgccaacc tgacccagga aaccaaggtc ctgctggccg acatgatcaa cgaggaaatg 600
aacgacctgg ccaaccagat gatcgacttc gagatccccc tgggcgaccc cagggaccag 660
aagcagtacc agcaccagaa gtgcttccag gaatttgccc actgctacct ggtgaagtac 720
aagaccacca agggctggcc tagcagcacc gtgatcgccg accagtgccc cctgcccggc 780
aaccacccca ccgtgcagta cgcccaccag aacatctggg actactacgt gcccttcgag 840

cagatcaggc ccgagggctg gaacagcaag agctactacg aggacgcccg gatcggcggc 900
ttctacatcc ccaagtggct gcggaacaac agctacaccc acgtgctgtt ctgcagcgac 960
cagatctacg gcaagtggta caacatcgac ctgaccgccc aggaacggga gaacctgctg 1020
gtgcagaagc tgatcaacct ggccaagggc aacagcagcc agctgaagga cagagccatg 1080
cccgccgagt gggacaagca gggcaaggcc gacctgttcc ggcagatcaa caccctggac 1140
gtgtgcaacc ggcccgagat ggtgttcctg ctgaacagct cctactacga gttcagcctg 1200
tgggagggcg actgcggctt cacccggcag aacgtgaccc aggccaacag cctgtgcaag 1260
gacttctaca acaacagcaa gtggcagaag ctgcaccctt acagctgccg gttctggcgg 1320
tacaagcagg aaaaagaaga gaccaagtgc agcaacggcg agaagaagaa gtgcctgtac 1380
tacccccagt gggacacccc tgaggccctg tacgacttcg gcttcctggc ctacctgaac 1440
agcttcccca gccccatctg catcaagaac cagaccatcc gggagcccga gtacgagatc 1500
agcagcctgt acctggaatg catgaacgcc agcgaccggc acggcatcga cagcgccctg 1560
ctggccctga aaaccttcct gaacttcacc ggccagagcg tgaacgagat gcccctggcc 1620
agggccttcg tgggcctgac cgacccccaag ttcccccccca cctaccccaa catcacccgg 1680
gagagcagcg gctgcaacaa caacaagcgg aagcggcgga gcgtgaacaa ctacgagcgg 1740
ctgcggagca tgggctacgc tctgacaggc gccgtgcaga ccctgagcca gatcagcgac 1800
atcaacgacg agaggctgca gcacggcgtg tacctgctgc gggaccacgt ggtgaccctg 1860
atggaagccg ccctgcacga cgtgagcatc atggaaggca tgctggccat tcagcacgtg 1920
cacacccacc tgaatcacct gaaaaccatg ctgctgatga ggaagatcga ttggaccttc 1980
atcagaagcg actggatcca gcagcagctg cagaaaaccg acgacgagat gaagctgatc 2040
cggcggaccg cccggtccct ggtgtactac gtgacacaga ccagcagctc ccccaccgcc 2100
acaagctggg agatcggcat ctactacgag atcgtgatcc ccaagcacat ctacctgaat 2160
aactggcagg tgatcaacgt gggccacctg ctggaaagcg ccggacacct gacccacgtg 2220
aaggtgaaac acccctacga gatcatcaac aaagagtgca gcgacaccca gtacctgcac 2280
ctggaagagt gcatccggga ggactacgtg atctgcgaca tcgtgcagat tgtgcagccc 2340
tgcggcaatg ccaccgagct gtccgactgc cccgtgaccg ccctgaaagt gaaaaccccc 2400
tacatccagg tgtccccccct gaagaacggc tcctacctgg tgctgtccag caccaaggac 2460
tgcagcatcc ccgcctacgt gcccagcgtg gtgaccgtga atgagaccgt gaagtgcttc 2520
ggcgtggagt tccacaagcc cctgtacgcc gagaccaaga ccagctacga gccccaggtg 2580
ccacacctga agctgcggct gccccacctg accggcatca tcgccagcct gcagagcctg 2640
gaaatcgagg tgaccagcac ccaggaaaac atcaaggacc agatcgagcg ggccaaggcc 2700

cagctgctga ggctggacat ccacgagggc gacttccccg actggctgaa gcaggtggcc 2760

agcgccacca gggacgtgtg gcctgccgcc gccagcttca tccagggcgt gggcaacttc 2820

ctgagcaaca ccgcccaggg catcttcggc agcgccgtga gcctgctgtc ctacgccaag 2880

cccatcctga tcggcatcgg cgtgattctg ctgatcgccc tgctgttcaa gatcatcagc 2940

tggctgcctg gcaagcccaa gaagaactga 2970

<210> 11
<211> 2970
<212> DNA
<213> Foamyvirus

<400> 11

atggcccctc ccatgaccct ggaacagtgg ctgctgtggc ggcggatgag ccaggcccac 60

caggccctgg aaaacgtgac caccctgacc gaggaacagc ggcagcaggt gatcatcgac 120

atccagcacg aggacgtggt gcccacccgg atggaccggc tgcggtacct ggcctacagc 180

tgctgcgcca cctccacccg ggtgctgtgc tggatcgtgc tggtgtgcgt gctgctgctg 240

gtggtgttca tcagctgctt cgtgaccatg agccggatcc agtggaacaa ggacatcgcc 300

gtgttcggcc ccgtgatcga ctggaacgtg agccagcagg ccgtgatcca gcagatccgg 360

gccaagcggc tggccagatc catccgggtc gagcacgcca ccgagaccta cgtggaggtg 420

aacatgacca gcatccccca gggcgtgctg tacgtgcccc accccgagcc catcatcctg 480

aaagaacggg tgctgggcct gagccaggtg atcatgatca acagcgagaa catcgccaac 540

accgccaacc tgacccagga aaccaaggtc ctgctggccg acatgatcaa cgaggaaatg 600

aacgacctgg ccaaccagat gatcgacttc gagatccccc tgggcgaccc cagggaccag 660

aagcagtacc agcaccagaa gtgcttccag gaatttgccc actgctacct ggtgaagtac 720

aagaccacca agggctggcc tagcagcacc gtgatcgccg accagtgccc cctgcccggc 780

aaccacccca ccgtgcagta cgcccaccag aacatctggg actactacgt gcccttcgag 840

cagatcaggc ccgagggctg gaacagcaag agctactacg aggacgcccg gatcggcggc 900

ttctacatcc ccaagtggct gcggaacaac agctacaccc acgtgctgtt ctgcagcgac 960

cagatctacg gcaagtggta caacatcgac ctgaccgccc aggaacggga gaacctgctg 1020

gtgcagaagc tgatcaacct ggccaagggc aacagcagcc agctgaagga cagagccatg 1080

cccgccgagt gggacaagca gggcaaggcc gacctgttcc ggcagatcaa caccctggac 1140

gtgtgcaacc ggcccgagat ggtgttcctg ctgaacagct cctactacga gttcagcctg 1200

tgggagggcg actgcggctt cacccggcag aacgtgaccc aggccaacag cctgtgcaag 1260

gacttctaca acaacagcaa gtggcagaag ctgcaccctt acagctgccg gttctggcgg 1320

tacaagcagg aaaaagaaga gaccaagtgc agcaacggcg agaagaagaa gtgcctgtac 1380

tacccccagt gggacacccc tgaggccctg tacgacttcg gcttcctggc ctacctgaac 1440
agcttcccca gccccatctg catcaagaac cagaccatcc gggagcccga gtacgagatc 1500
agcagcctgt acctggaatg catgaacgcc agcgaccggc acggcatcga cagcgccctg 1560
ctggccctga aaaccttcct gaacttcacc ggccagagcg tgaacgagat gcccctggcc 1620
agggccttcg tgggcctgac cgaccccaag ttccccccca cctaccccaa catcacccgg 1680
gagagcagcg gctgcaacaa caacaagcgg aagcggcgga gcgtgaacaa ctacgagcgg 1740
ctgcggagca tgggctacgc tctgacaggc gccgtgcaga ccctgagcca gatcagcgac 1800
atcaacgacg agaggctgca gcacggcgtg tacctgctgc gggaccacgt ggtgaccctg 1860
atggaagccg ccctgcacga cgtgagcatc atggaaggca tgctggccat tcagcacgtg 1920
cacacccacc tgaatcacct gaaaaccatg ctgctgatga ggaagatcga ttggaccttc 1980
atcagaagcg actggatcca gcagcagctg cagaaaaccg acgacgagat gaagctgatc 2040
cggcggaccg cccggtccct ggtgtactac gtgacacaga ccagcagctc ccccaccgcc 2100
acaagctggg agatcggcat ctactacgag atcgtgatcc ccaagcacat ctacctgaat 2160
aactggcagg tgatcaacgt gggccacctg ctggaaagcg ccggacacct gacccacgtg 2220
aaggtgaaac acccctacga gatcatcaac aaagagtgca gcgacaccca gtacctgcac 2280
ctggaagagt gcatccggga ggactacgtg atctgcgaca tcgtgcagat tgtgcagccc 2340
tgcggcaatg ccaccgagct gtccgactgc cccgtgaccg ccctgaaagt gaaaaccccc 2400
tacatccagg tgtcccccct gaagaacggc tcctacctgg tgctgtccag caccaaggac 2460
tgcagcatcc ccgcctacgt gcccagcgtg gtgaccgtga atgagaccgt gaagtgcttc 2520
ggcgtggagt tccacaagcc cctgtacgcc gagaccaaga ccagctacga gccccaggtg 2580
ccacacctga agctgcggct gccccacctg accggcatca tcgccagcct gcagagcctg 2640
gaaatcgagg tgaccagcac ccaggaaaac atcaaggacc agatcgagcg ggccaaggcc 2700
cagctgctga ggctggacat ccacgagggc gacttccccg actggctgaa gcaggtggcc 2760
agcgccacca gggacgtgtg gcctgccgcc gccagcttca tccagggcgt gggcaacttc 2820
ctgagcaaca ccgcccaggg catcttcggc agcgccgtga gcctgctgtc ctacgccaag 2880
cccatcctga tcggcatcgg cgtgattctg ctgatcgccc tgctgttcaa gatcatcagc 2940
tggctgcctg gccggccccg gcggaactga 2970

<210> 12
<211> 2970
<212> DNA
<213> Foamyvirus

<400> 12

atggcccctc ccatgacccct ggaacagtgg ctgctgtggc ggcggatgag ccaggcccac 60

```
caggccctgg aaaacgtgac caccctgacc gaggaacagc ggcagcaggt gatcatcgac     120
atccagcacg aggacgtggt gcccacccgg atggaccggc tgcggtacct ggcctacagc     180
tgctgcgcca cctccacccg ggtgctgtgc tggatcgtgc tggtgtgcgt gctgctgctg     240
gtggtgttca tcagctgctt cgtgaccatg agccggatcc agtggaacaa ggacatcgcc     300
gtgttcggcc ccgtgatcga ctggaacgtg agccagcagg ccgtgatcca gcagatccgg     360
gccaagcggc tggccagatc catccgggtc gagcacgcca ccgagaccta cgtggaggtg     420
aacatgacca gcatccccca gggcgtgctg tacgtgcccc accccgagcc catcatcctg     480
aaagaacggg tgctgggcct gagccaggtg atcatgatca acagcgagaa catcgccaac     540
accgccaacc tgacccagga aaccaaggtc ctgctggccg acatgatcaa cgaggaaatg     600
aacgacctgg ccaaccagat gatcgacttc gagatccccc tgggcgaccc cagggaccag     660
aagcagtacc agcaccagaa gtgcttccag gaatttgccc actgctacct ggtgaagtac     720
aagaccacca agggctggcc tagcagcacc gtgatcgccg accagtgccc cctgcccggc     780
aaccaccccca ccgtgcagta cgcccaccag aacatctggg actactacgt gcccttcgag     840
cagatcaggc ccgagggctg gaacagcaag agctactacg aggacgcccg gatcggcggc     900
ttctacatcc ccaagtggct gcggaacaac agctacaccc acgtgctgtt ctgcagcgac     960
cagatctacg gcaagtggta caacatcgac ctgaccgccc aggaacggga gaacctgctg    1020
gtgcagaagc tgatcaacct ggccaagggc aacagcagcc agctgaagga cagagccatg    1080
cccgccgagt gggacaagca gggcaaggcc gacctgttcc ggcagatcaa caccctggac    1140
gtgtgcaacc ggcccgagat ggtgttcctg ctgaacagct cctactacga gttcagcctg    1200
tgggagggcg actgcggctt cacccggcag aacgtgaccc aggccaacag cctgtgcaag    1260
gacttctaca acaacagcaa gtggcagaag ctgcaccctt acagctgccg gttctggcgg    1320
tacaagcagg aaaaagaaga gaccaagtgc agcaacggcg agaagaagaa gtgcctgtac    1380
tacccccagt gggacacccc tgaggccctg tacgacttcg gcttcctggc ctacctgaac    1440
agcttcccca gccccatctg catcaagaac cagaccatcc gggagcccga gtacgagatc    1500
agcagcctgt acctggaatg catgaacgcc agcgaccggc acggcatcga cagcgccctg    1560
ctggccctga aaaccttcct gaacttcacc ggccagagcg tgaacgagat gcccctggcc    1620
agggccttcg tgggcctgac cgaccccaag ttccccccca cctaccccaa catcacccgg    1680
gagagcagcg gctgcaacaa caacaagcgg aagcggcgga gcgtgaacaa ctacgagcgg    1740
ctgcggagca tgggctacgc tctgacaggc gccgtgcaga ccctgagcca gatcagcgac    1800
atcaacgacg agaggctgca gcacggcgtg tacctgctgc gggaccacgt ggtgaccctg    1860
atggaagccg ccctgcacga cgtgagcatc atggaaggca tgctggccat tcagcacgtg    1920
cacacccacc tgaatcacct gaaaaccatg ctgctgatga ggaagatcga ttggaccttc    1980
```

```
atcagaagcg actggatcca gcagcagctg cagaaaaccg acgacgagat gaagctgatc    2040
cggcggaccg cccggtccct ggtgtactac gtgacacaga ccagcagctc ccccaccgcc    2100
acaagctggg agatcggcat ctactacgag atcgtgatcc ccaagcacat ctacctgaat    2160
aactggcagg tgatcaacgt gggccacctg ctggaaagcg ccggacacct gacccacgtg    2220
aaggtgaaac acccctacga gatcatcaac aaagagtgca gcgacaccca gtacctgcac    2280
ctggaagagt gcatccggga ggactacgtg atctgcgaca tcgtgcagat tgtgcagccc    2340
tgcggcaatg ccaccgagct gtccgactgc cccgtgaccg ccctgaaagt gaaaaccccc    2400
tacatccagg tgtccccct gaagaacggc tcctacctgg tgctgtccag caccaaggac    2460
tgcagcatcc ccgcctacgt gcccagcgtg gtgaccgtga atgagaccgt gaagtgcttc    2520
ggcgtggagt tccacaagcc cctgtacgcc gagaccaaga ccagctacga gccccaggtg    2580
ccacacctga agctgcggct gccccacctg accggcatca tcgccagcct gcagagcctg    2640
gaaatcgagg tgaccagcac ccaggaaaac atcaaggacc agatcgagcg ggccaaggcc    2700
cagctgctga ggctggacat ccacgagggc gacttccccg actggctgaa gcaggtggcc    2760
agcgccacca gggacgtgtg gcctgccgcc gccagcttca tccagggcgt gggcaacttc    2820
ctgagcaaca ccgcccaggg catcttcggc agcgccgtga gcctgctgtc ctacgcccgg    2880
cccatcctga tcggcatcgg cgtgattctg ctgatcgccc tgctgttccg gatcatcagc    2940
tggctgcctg gccggccccg gcggaactga                                     2970
```